# EUROPEAN PATENT APPLICATION

(11) **EP 3 971 214 A1**
(43) Date of publication of application: **23.03.2022**
(21) Application number: 20805380.1
(22) Date of filing: 03.01.2020
(51) Int. Cl.: C07K 19/00, C12N 15/62, C12P 21/00, A61K 38/26, A61P 3/04, A61P 3/00

(54) **FUSION PROTEIN FOR TREATMENT OF METABOLIC DISEASE**

(30) Priority: 16.05.2019 CN 201910407446
(71) Applicant: Zhejiang Doer Biologics Corporation, Hangzhou, Zhejiang 310018 (CN)
(72) Inventor: HUANG, Yanshan, Hangzhou, Zhejiang 310018 (CN)
(74) Representative: Canzler & Bergmeier Patentanwälte Partnerschaft mbB
(86) International application number: PCT/CN2020/070234
(87) International publication number: WO 2020/228360

(57) **Abstract**

The present disclosure relates to the field of biotechnology, and in particular, to a fusion protein for the treatment of metabolic diseases, a preparation method therefor and use thereof. The present disclosure provides a fusion protein, including a Glucagon analogue fragment and a long-acting protein unit fragment, the Glucagon analogue fragment including: a) a polypeptide fragment having an amino acid sequence shown in SEQ ID No. 81; or b) a polypeptide fragment that has an amino acid sequence having at least 90% sequence identity with SEQ ID NO. 81 and has a function of the polypeptide fragment defined in a). The fusion protein of the present disclosure has good stability and good hypoglycemic and weight loss effects for mice.

## Description

### Technical Field

The present disclosure relates to the field of biotechnology, and in particular, to a fusion protein for the treatment of metabolic diseases, a preparation method therefor and use thereof.

### Background

Diabetes can be divided into Type 1 diabetes and Type 2 diabetes according to pathological characteristics. Type 1 diabetes is mainly manifested by insufficient insulin secretion and requires daily insulin injections; Type 2 diabetes is caused by the inability of the body to effectively use insulin. Most of the diabetes patients are Type 2 diabetes patients. It is estimated that approximately 80-90% of patients with Type 2 diabetes are significantly obese (Center for disease control and prevention (CDC) National Diabetes Fact Sheet, 2014).

Medicines for treating Type 2 diabetes such as sulfonylureas and thiazolidinediones are effective in lowering glucose, but the main disadvantage is that they cause weight gain (N Engl J Med, 355(23):2427-43, 2006). Protein drugs for Type 2 diabetes are mainly GLP-1R (GLP-1 receptor) agonists, such as Dulaglutide (trade name: Trulicity^{®}), Albilutide (trade name: Tanzeum^{®}), Liraglutide (trade names: SAXENDA ^{®} and Victoza^{®}, used to treat obesity and diabetes, respectively), Exenatide (trade name: Byetta^{®}), Lixisenatide (trade name: Lyxumia^{®}) and Semaglutide. GLP-1R agonists have a significant hypoglycemic effect. Unlike insulin, the hypoglycemic effect of GLP-1R agonists is strictly blood glucose-dependent, which is less likely to cause hypoglycemia, and also has a weight loss effect. However, these drugs have side effects in the treatment, mainly gastrointestinal effects such as nausea, and most of the weight loss does not exceed 10% of the average body weight. Bariatric surgery, which can significantly ameliorate obesity and treat diabetes, is not widely used because most patients are unwilling to undergo such an operation due to the risk of surgery and long-term sequelae (Obesity and Diabetes, New Surgical and Nonsurgical Approaches, Springer Press, 2015).

Therefore, the new generation of diabetes drugs is mainly focused on the research of dual functional or multi-functional incretin receptor agonists, such as dual agonists, like GLP-1R/GCGR and GLP-1R/GIPR agonists, and even tri-agonist GLP-1R/GIPR/GCGR (Nature Medicine, 22 (7):694-695, 2016). Among them, the receptors for Glucagon (GCG) and GLP-1 (Glucagon-like peptide-1) are structurally related, but the two hormones show diametrically opposite effects in controlling glucose. Clinically, GLP-1 and its analogues are mainly used for controlling blood glucose in diabetics, while Glucagon (GCG) is for acute hypoglycemia. In recent years, emerging research suggests that Glucagon, despite its risk of raising blood glucose, can effectively reduce body weight. More importantly, GLP-1 and Glucagon seem to have a positive additive or synergistic physiological effect. For example, Glucagon receptor (GCGR) and GLP-1 receptor (GLP-1R) dual agonists are more effective than GLP-1R mono-agonists in losing weight. Although GCGR stimulation may cause blood glucose increase, this risk can be appropriately offset by GLP-1R stimulation and/or GIPR stimulation. The various forms of hybrid peptides currently in the clinical or preclinical researches are described in detail in the Review paper of Matthias H. Tschöp et al. (Cell Metab, 24(1):51-62, 2016). Alessandro Pocai et al. reported a GLP-1R/GCGR dual agonist based on Oxyntomodulin (OXM) (Diabetes; 58(10): 2258-2266, 2009), and Richard D. DiMarchi *et al.* reported a GCG-based GLP-1R/GCGR dual agonist (US9018164B2) and even GLP-1R/GCGR/GIPR tri-agonist (US9150632B2). Most of these multispecific hybrid peptides are based on GLP-1 or GCG with sequence mutations to enhance activity and resist protease hydrolysis, and most of these analogues mutate the serine (Ser) at the second position into an unnatural amino acid Aib to resist the enzymatic hydrolysis by DPP-IV. Taspoglutide, a GLP-1R agonist hypoglycemic drug developed by Roche in collaboration with Ipsen (with the introduction of the unnatural amino acid Aib), caused an anti-drug antibody positive rate of 49%, and all phase III clinical studies of Taspoglutide were eventually stopped (Diabetes Care, 36:498-504, 2013). There are also a few reports of Glucagon analogues conjugated to fatty acids with natural serine (Ser) at the second position (Diabetes ObesMetab, 18(12):1176-1190, 2016 ). This analogue (MEDI0382) includes 30 amino acids, and 9 amino acids were introduced compared to natural Glucagon. Also, although the natural Ser amino acid is retained at the second position of MEDI0382, it can only support a once-a-day dosing frequency. PEG conjugation or fatty acid conjugation are commonly used in order to reduce the dosing frequency, such as those used in Semaglutide. But conjugation process is complicated as chemical synthesis and cross-linking are needed.

As an important metabolic regulator, fibroblast growth factor 21 (FGF21) has been shown to improve a variety of metabolic abnormalities in preclinical animal models of Type 2 diabetes mellitus (T2DM). As a therapeutic drug for diabetic patients, FGF21 has potential effects in increasing insulin sensitivity, improving blood glucose control and weight loss, lowering low-density lipoprotein cholesterol (LDL-C) and triglycerides, and increasing high-density lipoprotein cholesterol (HDL-C) levels. In diabetic mice and monkeys, human FGF21 can reduce the concentrations of fasting serum glucose, fasting serum triglyceride, insulin and glucagon. In addition, FGF21induced dose-dependent overall weight loss in rodent models of diet-induced obesity. Therefore, FGF21 has a therapeutic potential in diseases such as diabetes, obesity, dyslipidemia and metabolic syndrome.

However, FGF21 has a very short serum half-life: 30 minutes in mice and 2 hours in monkeys. Therefore, to maintain biological activity *in vivo*, daily injection or continuous infusion of corresponding FGF21 protein is required. In human studies, circulating levels of FGF21 are elevated in patients with obesity, dyslipidemia, TDM2 and other diseases associated with insulin resistance, and some studies have shown that increased FGF21 concentrations are associated with increased risk of cerebrovascular disease (CVD), and that increased FGF21 concentrations also lead to osteoporosis and affect reproduction (promoting metabolism and thus leading to energy deficiency) (Proc Natl AcadSci USA, 109(8):3143-8, 2012; MolMetab, 5(8):690-8, 2016). The sequence homology of the FGF family and the wide distribution of FGFR1 receptors have also raised concerns about the potential safety issues brought by the high-dose clinical use of FGF21 (J Intern Med, 281(3):233-246, 2017).

GCGR/GLP-1R dual agonists, GLP-1R/GIPR dual agonists, GLP-1R/GCGR/GIPR tri-agonists and FGF21 analogues have all been used for the treatment of diabetes and obesity, respectively, and there are also reports of the preparation of GLP-1 analogues with FGF21 via Fc fusion to form dual functional proteins (The American Diabetes Association, 2016). As described above, dual functional peptides or even triple functional peptides based on Glucagon or Oxyntomodulin generally need to replace some amino acids with unnatural amino acids to improve stability and activity, and even need to be modified by fatty acids or PEG. It is technically extremely difficult to fuse and express these peptides with FGF21 analogues into a single molecule. There is also currently no report on the combined use of dual or tri-agonist polypeptides and FGF21 analogues.

### Summary

In view of the above-described shortcomings of the prior art, the present disclosure provides a fusion protein for the treatment of metabolic diseases, a preparation method therefor and use thereof, for solving the problems in the prior art.

To achieve the foregoing and other related purposes, a first aspect of the present disclosure provides a fusion protein for the treatment of metabolic diseases including a Glucagon analogue fragment and a long-acting protein unit fragment, the Glucagon analogue fragment including:

a) a polypeptide fragment having an amino acid sequence shown in SEQ ID No. 81:
X₁SX₃GTFTSDYSKYLDX₁₆X₁₇X₁₈AQDFVQWLX₂₇X₂₈X₂₉X^{z}(SEQ ID No. 81)
X₁ is selected from H or Y; X₃ is selected from Q or E; X₁₆ is selected from any amino acid except Y, N, W, and H; X₁₇ is selected from any amino acid except P, L, T, F and H; X₁₈ is selected from any amino acid except P, F, H and W; X₁₇ and X₁₈ are not R at the same time; X₂₇ is selected from M or L; X₂₈ is selected from D or A; X₂₉ is T or missing; X^{z} is selected from GGPSSGAPPPS or GPSSGAPPPS;
or b) a polypeptide fragment that has an amino acid sequence having at least 90% sequence identity with SEQ ID NO. 81 and has a function of the polypeptide fragment defined in a).

In some embodiments of the present disclosure, the polypeptide fragment in a) is selected from a polypeptide fragment having an amino acid sequence shown in one of SEQ ID NO. 29, SEQ ID NO. 32, SEQ ID NO. 33, SEQ ID NO. 35, SEQ ID NO. 38, SEQ ID NO. 42, SEQ ID NO. 43, and SEQ ID NO. 44.

In some embodiments of the present disclosure, the long-acting protein unit fragment is derived from the Fc portion of mammalian immunoglobulin.

In some embodiments of the present disclosure, the long-acting protein unit fragment includes:
c) a polypeptide fragment having an amino acid sequence shown in one of SEQ ID NO. 4-13; or
d) a polypeptide fragment that has an amino acid sequence having at least 90% sequence identity with one of SEQ ID NO. 4-13 and has a function of the polypeptide fragment defined in c).

In some embodiments of the present disclosure, the fusion protein further includes a first linker peptide fragment, the first linker peptide fragment being located between a Glucagon analogue fragment and a long-acting protein unit fragment. Preferably, the first linker peptide fragment is rich in G, S and/or A.

In some embodiments of the present disclosure, the first linker peptide fragment includes a polypeptide fragment having an amino acid sequence shown in one of SEQ ID NO. 14-23.

In some embodiments of the present disclosure, the fusion protein includes, in order from N-terminal to C-terminal, a Glucagon analogue fragment, a first linker peptide fragment and a long-acting protein unit fragment.

In some embodiments of the present disclosure, the amino acid sequence of the fusion protein is shown in one of SEQ ID NO.56, SEQ ID NO.59, SEQ ID NO.60, SEQ ID NO.62, SEQ ID NO.65, SEQ ID NO.69, SEQ ID NO.70, and SEQ ID NO.71.

In some embodiments of the present disclosure, the fusion protein further includes an FGF21 analogue fragment.

In some embodiments of the present disclosure, the FGF21 analogue fragment includes:
e) a polypeptide fragment having an amino acid sequence shown in SEQ ID NO. 119:

HPIPDSSPLLQFGGQVRQX19YLYTDDAQQTEX31HLEIX36EDGTVGX43AX45DQ
SPESLLQLX56ALKPGVIQILGVKTSRFLCQRPDGALYGSLHFDPEACSFREX98
LLEDGYNVYQSEAHGLPLHX118PGNX122SPHRDPAPRGPX134RFLPLPGLPPAL
PEPPGILAPQPPDVGSSDPLX167MVX170X171SQX174RSPSX179X180X181;

The N terminal HPIPDSS may be missing or partially missing; X₁₉ is selected from R, Y, V, E or C; X₃₁ is selected from A or C; X₃₆ is selected from R or K; X₄₃ is selected from G or C; X₄₅ is selected from A, K, E or V; X₅₆ is selected from K, R, V or I; X₉₈ is selected from L, R or D; X₁₁₈ is selected from L or C; X₁₂₂ is selected from K or R; X₁₃₄ is selected from A or C; X₁₆₇ is selected from S, A or R; X₁₇₀ is selected from G or E; X₁₇₁ is selected from P or G; X₁₇₄ is selected from G, A or L; X₁₇₉ is selected from Y, A or F; X₁₈₀ is selected from A or E; X₁₈₁ is selected from S, K or is missing;
or f) a polypeptide fragment that has an amino acid sequence having at least 80% sequence identity with SEQ ID NO. 119 and has a function of the polypeptide fragment defined in e).

In some embodiments of the present disclosure, the polypeptide fragment in e) is selected from a polypeptide fragment having an amino acid sequence shown in one of SEQ ID NO. 87-90.

In some embodiments of the present disclosure, the fusion protein further includes a second linker peptide fragment, the second linker peptide fragment being located between a long-acting protein unit fragment and an FGF21 analogue fragment. Preferably, the second linker peptide fragment is rich in G, S and/or A.

In some embodiments of the present disclosure, the second linker peptide fragment includes a polypeptide fragment having an amino acid sequence shown in one of SEQ ID NO. 14-23.

In some embodiments of the present disclosure, the fusion protein includes, in order from N-terminal to C-terminal, a Glucagon analogue fragment, a first linker peptide fragment, a long-acting protein unit fragment and an FGF21 analogue fragment;
or, the fusion protein includes, in order from N-terminal to C-terminal, a Glucagon analogue fragment, a first linker peptide fragment, a long-acting protein unit fragment, a second linker peptide fragment and an FGF21 analogue fragment.

In some embodiments of the present disclosure, the amino acid sequence of the fusion protein is shown in one of SEQ ID NO. 91-115.

A second aspect of the present disclosure provides an isolated polynucleotide encoding the fusion protein.

A third aspect of the present disclosure provides a construct, the construct including the isolated polynucleotide.

A fourth aspect of the present disclosure provides an expression system, and the expression system includes the construct or incorporates the exogenous polynucleotide in the genome.

A fifth aspect of the present disclosure provides a method for preparing the fusion protein, including: culturing the expression system under suitable conditions to express the fusion protein, and isolating and purifying to provide the fusion protein.

A sixth aspect of the present disclosure provides a pharmaceutical composition, which includes: the fusion protein, or culture of the expression system.

A seventh aspect of the present disclosure provides the use of the fusion protein and the pharmaceutical composition in the preparation of a drug.

The drug described in seventh aspect of the present disclosure is selected from drugs for the treatment of metabolism-related diseases.

### Brief Description of the Drawings

FIG. 1A: the result of serum stability over time.
FIG. 1B: the result of serum stability over time.
FIG. 2A: schematic diagram of the hypoglycemic effect of the fusion proteins in Embodiment 6 on db/db mice.
FIG. 2B: schematic diagram of the hypoglycemic effect of the fusion proteins in Embodiment 6 on db/db mice.
FIG. 3: schematic diagram of the effect of the fusion protein in Embodiment 7 on the body weight of DIO mice.

### Detailed Description of the Preferred Embodiments

Based on extensive exploration and research, the inventors of the present disclosure unexpectedly found that after fusing GCG analogues with F_{C}, the GLP-1R and GCGR agonism activities were completely different, thus providing a new fusion protein. The fusion protein has good stability and good hypoglycemic and weight loss effects in mice, thus having good prospects for industrialization. The present disclosure was completed on this basis.

In the present disclosure, the term "diabetes" usually includes Type 1 diabetes, Type 2 diabetes, gestational diabetes, and other symptoms that cause hyperglycemia. This term can be used for describing metabolic disorders, in which the pancreas cannot produce enough insulin, or the somatic cells fail to respond to insulin properly, which leads to a decrease in glucose absorption efficiency of tissue cells and causes glucose to accumulate in the blood. Type 1 diabetes, also known as insulin-dependent diabetes and juvenile-onset diabetes, is caused by the destruction of β cells and usually results in absolute insulin deficiency. Type 2 diabetes, also known as non-insulin-dependent diabetes and adult-onset diabetes, is generally associated with insulin resistance.

In the present disclosure, the term "obesity" refers to excess fat tissue, caused by excess calories stored in fat when energy intake exceeds energy consumption. In the present disclosure, obesity is optimally regarded as the formation of excess adipose tissue to any degree that is hazardous to health. In the present disclosure, individuals with a body mass index (BMI = body weight (kg) divided by the square of height (m)) exceeding 25 are considered obese.

In the present disclosure, "Incretin" is a gastrointestinal hormone that regulates blood glucose by enhancing glucose-stimulated insulin secretion (also known as glucose-dependent insulin secretion, GSIS) (Lancet, 368:1696-705, 2006). Incretin can also slow down the rate of nutrient absorption by delaying gastric emptying, and directly reduce food absorption. At the same time, incretin can also inhibit intestinal α cells from secreting Glucagon. Hitherto, there are two known types of incretin: glucagon-like peptide-1 (GLP-1) and Glucose-dependent insulinotropic polypeptide (GIP).

In the present disclosure, "GIP" usually refers to a 42-amino acid peptide obtained by the proteolytic processing of 133-amino acid precursor (pre-pro-GIP). These molecules are involved in various biological functions, including glucose homeostasis, insulin secretion, gastric emptying and intestinal growth, and food intake regulation.

In the present disclosure, "glucagon-like peptide" (GLP-1) is a 30- or 31-amino acid polypeptide incretin hormone secreted from intestinal L-cells. GLP-1 includes two active forms: GLP-1 (7-36) and GLP-1 (7-37). GLP-1 is released into the circulation after a meal, and exerts its biological activity by activating GLP-1 receptors. GLP-1 has various biological effects, including glucose-dependent insulin secretion, inhibition of glucagon production, delay of gastric emptying, and appetite suppression (Trends PharmacolSci, 32(1):8-15, 2011). Natural GLP-1 is rapidly degraded by dipeptidyl peptidase-4 (DPP- IV), neutral endopeptidase (NEP), plasma kallikrein or plasmin, which limits its therapeutic potential. Since natural GLP-1 has an ultra short half-life of only about 2 min in *vivo*, methods have emerged to improve efficacy by using chemical modifications and/or formulation forms to treat diabetes and obesity (Bioorg Med Chem Lett, 23(14): 4011-8, 2013; Expert OpinInvestig Drugs, 25(2):145-58, 2016).

In the present disclosure, "Glucagon" is a 29-amino acid peptide, which corresponds to amino acids at position 53-81 of prepro Glucagon, and the sequence is shown in SEQ ID NO: 24 (Nutrition, Metabolism & Cardiovascular Diseases, 16: S28-S34, 2006). Glucagon receptor activation has been shown to increase energy consumption and reduce food intake in both rodents and humans (Nat. Rev. Endocrinol, 6: 689-697, 2010), and the effects are stable and persistent in rodents. Glucagon has many physiological effects, such as increasing blood glucose levels in hypoglycemic conditions by stimulating glycogen breakdown and gluconeogenesis, regulating the production of liver ketone, regulating the bile acid metabolism and vagus nerve-through satiety effects. In treatment, Glucagon has been used for acute hypoglycemia. Glucagon receptor activation reduces food intake, and promotes fat breakdown and weight loss in animals and humans.

In the present disclosure, the term "receptor agonist" can usually be defined as a polypeptide, protein, or other small molecules that bind to a receptor and trigger the usual response of a natural ligand.

In the present disclosure, "GLP-1 receptor (GLP-1R) agonist" can usually be defined as a polypeptide, protein, or other small molecules that bind to GLP-1R and can initiate the same or similar characteristic response as natural GLP-1. GLP-1R agonists activate GLP-1R completely or partially, and then cause a series of downstream signaling pathway reactions inside the cell to produce corresponding cell activity, such as β cells secreting insulin. Typical GLP-1R agonists include natural GLP-1 and mutants and analogues thereof, such as Exenatide, Liraglutide and the like.

In the present disclosure, "GLP-1 analogues" or "GLP-1 mutants" all mean GLP-1R agonists and may be used interchangeably.

In the present disclosure, "Glucagon receptor (GCGR) agonist" may be defined as a polypeptide, protein or other small peptides that bind to GCGR and can initiate the same or similar characteristic response as natural Glucagon. GCGR agonists activate GCGR completely or partially, and then cause a series of downstream signaling pathway reactions inside the cell to produce corresponding cell activity, such as glycogenolysis of hepatocytes, gluconeogenesis, fatty acid oxidation and ketogenesis and the like.

In the present disclosure, "Glucagon analogues", "GCG analogues", "Glucagon mutants" and "GCG mutants" all mean Glucagon receptor agonists and may be used interchangeably.

In the present disclosure, "FGF21" usually refers to fibroblast growth factor (FGF), also known as a heparin-binding growth factor, which is a peptide substance secreted mainly by the pituitary and hypothalamus. FGF has many functions, such as promoting the mitosis of fibroblasts, promoting the growth of mesoderm cells, and stimulating the formation of blood vessels. FGF21 is an important member of the FGF family, and this hormone is currently being developed as a drug for obesity as well as for the treatment of diabetes, and the drug is already in clinical trials. FGF21 exerts its physiological effects through FGF21-related receptors (e.g. FGFR1c) and its co-receptor β-klotho (KLB).

In the present disclosure, "dimer" is formed by the natural non-covalent and covalent action of the constant region (F_{C}) of the immunoglobulin. If not otherwise specified, the dimers formed by F_{C} are all homodimers, as described in the dimers provided by the present disclosure.

In the present disclosure, "EC₅₀" (concentration for 50% of maximal effect) refers to the concentration required for a drug or substance to stimulate 50% of its corresponding biological response. The lower the EC₅₀ value, the stronger the stimulating or activation ability of the drug or substance. More intuitively, for example, the stronger the intracellular signal caused, the better the ability to induce the production of a hormone.

In the present disclosure, "low-density lipoprotein" (LDL), which is usually a type of plasma lipoprotein, is the main carrier of cholesterol in the blood and tends to deposit cholesterol on arterial walls. Leukocytes try to digest LDL, but this process turns them into toxins. More and more leukocytes are attracted to the area where the changes occur, resulting in possible inflammation of the arterial walls. Over time, as the process continues, these plaque deposits can accumulate in the arterial walls, making the channels very narrow and lacking in toughness. If too much plaque accumulates, the artery may be completely blocked. When the complex(LDL-C) formed by LDL and cholesterol creates too much plaque in the artery walls, blood will not be able to flow freely through the arteries. Plaque can suddenly collapse in the arteries at any time, causing a blockage in the blood vessels and eventually leading to heart disease.

In the present disclosure, "high-density protein" (HDL) usually helps to remove LDL from the arteries, acting as a scavenger to remove LDL from the arteries and return it to the liver.

In the present disclosure, "triglyceride (TG)" usually refers to another type of fat that is used to store excess energy from the diet. High levels of triglycerides in the blood are associated with atherosclerosis. High triglycerides can be caused by overweight and obesity, physical inactivity, smoking, excessive alcohol consumption and high carbohydrate (more than 60% of total calories) intake. Sometimes the underlying or genetic disease is the cause of high triglycerides. People with high triglycerides usually have high total cholesterol levels, including high LDL cholesterol and low HDL cholesterol, and many people with heart disease or diabetes also have high triglyceride levels.

The first aspect of the present disclosure provides a fusion protein, including a Glucagon analogue fragment and a long-acting protein unit fragment, the Glucagon analogue fragment including:

a) a polypeptide fragment having an amino acid sequence shown in SEQ ID No. 81:
X₁SX₃GTFTSDYSKYLDX₁₆X₁₇X₁₈AQDFVQWLX₂₇X₂₈X₂₉X^{z} (SEQ ID No. 81)
X₁ is selected from H or Y; X₃ is selected from Q or E; X₁₆ is selected from any amino acid except Y, N, W, and H; X₁₇ is selected from any amino acid except P, L, T, F and H; X₁₈ is selected from any amino acid except P, F, H and W; X₁₇ and X₁₈ are not R at the same time; X₂₇ is selected from M or L; X₂₈ is selected from D or A; X₂₉ is T or missing; X^{z} is selected from GGPSSGAPPPS (SEQ ID NO. 2) or GPSSGAPPPS (SEQ ID NO. 3);
or b) a polypeptide fragment that has an amino acid sequence having at least 90% sequence identity with SEQ ID NO. 81 and has a function of the polypeptide fragment defined in a). Specifically, the amino acid sequence in b) refers to: a polypeptide fragment obtained by substituting, deleting or adding one or more (specifically 1- 50,1- 30, 1- 20, 1- 10, 1-5, 1-3, 1, 2 or 3) amino acids in an amino acid sequence as shown in SEQ ID No.81, or by adding one or more (specifically 1-50,1- 30, 1- 20, 1- 10, 1-5, 1-3, 1, 2 or 3) amino acids to the N-terminal and/or C-terminal of an amino acid sequence as shown in SEQ ID No.81, and having a function of a polypeptide fragment as shown in SEQ ID No.81, for example, a function of GCGR/GLP-1R dual agonist activity, GLP-1R/GIPR dual agonist activity or GLP-1R/GCGR/GIPR tri-agonist activity, and being resistant to *in vivo* protease hydrolysis. The amino acid sequence in b) may have at least 90%, 93%, 95%, 97%, or 99% sequence identity with SEQ ID No. 81. In a specific embodiment of the present disclosure, the polypeptide fragment in a) may be a polypeptide fragment having an amino acid sequence shown in one of SEQ ID NO. 29, SEQ ID NO. 32, SEQ ID NO. 33, SEQ ID NO. 35, SEQ ID NO. 38, SEQ ID NO. 42, SEQ ID NO. 43, and SEQ ID NO. 44. The present disclosure found that the GCG analogues obtained by the screening of the present disclosure are stable enough to support a once-a-week dosing frequency after fusion with F_{C}, reducing the potential risk of immunogenicity, even if the natural Ser at the second position is retained. Modifications at positions 16, 17 and 18, in addition to attenuating the degradation of GCG analogues by endopeptidases, also resulted in better maintenance of GCGR agonist activity.

In the fusion protein of the present disclosure, the long-acting protein unit fragment may be derived from the F_{C} portion of mammalian immunoglobulin. The immunoglobulin is a polypeptide chain molecule containing a disulfide bond, and generally includes two light chains and two heavy chains. The F_{C} portion of the immunoglobulin used herein has the usual meaning of terms as used in the field of immunology. Specifically, the term F_{C} portion refers to an antibody fragment obtained by removing two antigen-binding regions (Fab fragments) from an antibody. The F_{C} portion may include a hinge region, CH2 and CH3 domains. The F_{C} portion may further include one or more glycosylation sites. There are five kinds of human immunoglobulins with different effect characteristics and pharmacokinetic characteristics in humans: IgG, IgA, IgM, IgD and IgE. IgG is the most abundant immunoglobulin in serum. IgG also has the longest serum half-life in all immunoglobulins (about 23 days). Further, the long-acting protein unit fragment may be selected from a complete F_{C} portion of an immunoglobulin, a fragment of an F_{C} portion of an immunoglobulin, or a mutant of an Fc portion of an immunoglobulin. The F_{C} portion of the immunoglobulin used in the present disclosure may be derived from an F_{C} region of mammal IgG1, IgG2 or IgG4, or a mutant thereof. Preferably, the F_{C} portion of the immunoglobulin may be derived from an F_{C} region of human IgG1, IgG2 or IgG4, or a mutant thereof. More preferably, the Fc portion of the immunoglobulin may be derived from an F_{C} region of human IgG1 or IgG4, or a mutant thereof. In a preferred embodiment, position 297 of the Fc domain is replaced with glycine or alanine. The above content is based on the EU index number of kabat (Sequences of proteins of immunological interest, fifth edition, public health service, National Institutes of Health, Bethesda, MD (1991)).

In the fusion protein of the present disclosure, the long-acting protein unit fragment may include: c) a polypeptide fragment having an amino acid sequence shown in one of SEQ ID NO. 4-13; or d) a polypeptide fragment that has an amino acid sequence having at least 90% sequence identity with one of SEQ ID NO. 4-13 and has a function of the polypeptide fragment defined in c). Specifically, the amino acid sequence in d) refers to: a polypeptide fragment obtained by substituting, deleting or adding one or more(specifically 1- 50,1- 30, 1- 20, 1-10, 1-5, 1-3, 1, 2 or 3) amino acids in an amino acid sequence as shown in one of SEQ ID No.4-13, or by adding one or more (specifically 1- 50,1- 30, 1- 20, 1- 10, 1-5, 1-3, 1, 2 or 3) amino acids to the N-terminal and/or C-terminal of an amino acid sequence as shown in any one of SEQ ID No.4-13, and having a function of a polypeptide fragment as shown in one of SEQ ID No.4-13, for example, a function of improving the overall DPP-IV resistance of the fusion protein. The amino acid sequence in d) may have at least 90%, 93%, 95%, 97%, or 99% sequence similarity with one of No. 4-13.In a preferred embodiment of the present disclosure, the Fc domain may be derived from human IgG1 and is shown in SEQ ID NO. 12 or SEQ ID NO. 13. In another preferred embodiment of the present disclosure, the Fc domain may be derived from human IgG4 and is shown in SEQ ID NO. 9. The K at the terminal of the Fc chain may be removed, which will improve the uniformity of the expression product, as shown in SEQ ID NO. 5 or SEQ ID NO.9. The preferred GCG analogues of the present disclosure can obtain extremely high DPP-IV resistance when fused with Fc. There is no prior art revealing that the DPP-IV enzyme resistance of GCG analogues can be increased by fusing F_{C}, and pharmacodynamic experiments have shown stability to support a once-a-week dosing frequency. It is unexpected that this effect can be achieved by fusion. The retention of the Ser at the second position reduces the risk of immunogenicity on the one hand and maximizes the agonist activity of GCGR for effective weight loss on the other hand.

The fusion protein of the present disclosure may further include a first linker peptide fragment, the first linker peptide fragment being usually located between a Glucagon analogue fragment and a long-acting protein unit fragment. The first linker peptide fragment is usually rich in G, S and/or A. For example, the first linker peptide fragment may be composed of glycine G, serine S and alanine A. The person skilled in the art may select a suitable ratio of the content of G, S and A, preferably 12:3:1, 12:1:3, 12:1:2, or 12:2:1, and S or A may be missing. For example, the ratio of G to A may be 4:1 or the ratio of G to S may be 4:1. In a specific embodiment of the present disclosure, the first linker peptide fragment includes a polypeptide fragment having an amino acid sequence shown in one of SEQ ID NO. 14-23.

In the fusion protein of the present disclosure, the fusion protein includes, in order from N-terminal to C-terminal, a Glucagon analogue fragment, a first linker peptide fragment and a long-acting protein unit fragment. In a specific embodiment of the present disclosure, the amino acid sequence of the fusion protein is shown in one of SEQ ID NO.56, SEQ ID NO.59, SEQ ID NO.60, SEQ ID NO.62, SEQ ID NO.65, SEQ ID NO.69, SEQ ID NO.70, and SEQ ID NO.71.

In the fusion protein of the present disclosure, the fusion protein further includes an FGF21 analogue fragment, and the FGF21 analogue fragment may include:

e) a polypeptide fragment having an amino acid sequence shown in SEQ ID NO. 119:
HPIPDSSPLLQFGGQVRQX₁₉YLYTDDAQQTEX₃₁HLEIX₃₆EDGTVGX₄₃AX₄₅DQ SPESLLQLX₅₆ALKPGVIQILGVKTSRFLCQRPDGALYGSLHFDPEACSFREX₉₈ LLEDGYNVYQSEAHGLPLHX₁₁₈PGNX₁₂₂SPHRDPAPRGPX₁₃₄RFLPLPGLPPAL PEPPGILAPQPPDVGS SDPLX₁₆₇MVX₁₇₀X₁₇₁SQX₁₇₄RSPSX₁₇₉X₁₈₀X₁₈₁;
The N terminal HPIPDSS may be missing or partially missing; X₁₉ is selected from R, Y, V, E or C; X₃₁ is selected from A or C; X₃₆ is selected from R or K; X₄₃ is selected from G or C; X₄₅ is selected from A, K, E or V; X₅₆ is selected from K, R, V or I; X₉₈ is selected from L, R or D; X₁₁₈ is selected from L or C; X₁₂₂ is selected from K or R; X₁₃₄ is selected from A or C; X₁₆₇ is selected from S, A or R; X₁₇₀ is selected from G or E; X₁₇₁ is selected from P or G; X₁₇₄ is selected from G, A or L; X₁₇₉ is selected from Y, A or F; X₁₈₀ is selected from A or E; X₁₈₁ is selected from S, K or is missing;
or f) a polypeptide fragment that has an amino acid sequence having at least 90% sequence identity with SEQ ID NO. 119 and has a function of the polypeptide fragment defined in e). Specifically, the amino acid sequence in f) refers to: a polypeptide fragment obtained by substituting, deleting or adding one or more(specifically 1-50, 1-30, 1-20, 1-10, 1-5, 1-3, 1, 2 or 3) amino acids in an amino acid sequence as shown in SEQ ID No.119, or by adding one or more (specifically 1-50, 1-30, 1-20, 1-10, 1-5, 1-3, 1, 2 or 3) amino acids to the N-terminal and/or C-terminal of an amino acid sequence as shown in SEQ ID No.119, and having a function of a polypeptide fragment as shown in SEQ ID No.119, for example, the polypeptide fragment may be a polypeptide fragment having the same or similar biological function as natural FGF21 (SEQ ID NO. 87). The amino acid sequence in f) may have at least 80%, 85%, 90%, 93%, 95%, 97%, or 99% sequence identity with SEQ ID No. 119. The amino acid sequence of the FGF21 analogue fragment may be selected from the amino acid sequences of FGF21 analogues or mutants described in patents or applications such as US 20140213512, US 8188040, US 9493530, WO 2016114633, US 20150291677, US 9422353, US 8541369, US 7622445, US 7576190, US 20070142278, US 9006400 or US 20130252884. In a specific embodiment of the present disclosure, the polypeptide fragment in e) is selected from a polypeptide fragment having an amino acid sequence shown in one of SEQ ID NO. 87-90.

The fusion protein of the present disclosure may further include a second linker peptide fragment, the second linker peptide fragment being usually located between a long-acting protein unit fragment and an FGF21 analogue fragment. The second linker peptide fragment is usually rich in G, S and/or A. For example, the second linker peptide fragment may be composed of glycine G, serine S and alanine A. The person skilled in the art may select a suitable ratio of the content of G, S and A, preferably 12:3:1, 12:1:3, 12:1:2, or 12:2:1, and S or A may be missing. For example, the ratio of G to A may be 4:1 or the ratio of G to S may be 4:1. In a specific embodiment of the present disclosure, the second linker peptide fragment includes a polypeptide fragment having an amino acid sequence shown in one of SEQ ID NO. 14-23.

In the fusion protein of the present disclosure, the fusion protein may include, in order from N-terminal to C-terminal, a Glucagon analogue fragment, a first linker peptide fragment, a long-acting protein unit fragment and an FGF21 analogue fragment, or the fusion protein may include, in order from N-terminal to C-terminal, a Glucagon analogue fragment, a first linker peptide fragment, a long-acting protein unit fragment, a second linker peptide fragment and an FGF21 analogue fragment. In a specific embodiment of the present disclosure, the amino acid sequence of the fusion protein is shown in one of SEQ ID NO. 91-115. In an *in vivo* animal pharmacodynamic embodiment of the present disclosure, the fusion protein group described with Formula II (A352L1F6L5M2) has a more significant weight loss effect compared to the co-administered group of the fusion protein described with Formula I (A352L1F6) + FGF21 analogue (F6L5M2) in the same dose. The fusion protein A352L1F6L5M2 was shown to have potentially lower side effects and improved safety (Embodiment 7).

A second aspect of the present disclosure provides an isolated polynucleotide encoding the fusion protein of the first aspect.

A third aspect of the present disclosure provides a construct, which includes the isolated polynucleotide provided by the second aspect of the present disclosure. The construct may generally be obtained by inserting the isolated polynucleotide into a suitable expression vector. Those skilled in the art may select a suitable expression vector. For example, the expression vector may include but not limited to pcDNA3.1, pBudCE4.1, pEE14.1, pPIC9, etc.

A fourth aspect of the present disclosure provides an expression system, which includes the construct provided by the third aspect of the present disclosure or incorporates the exogenous polynucleotide provided by the second aspect of the present disclosure in the genome. The expression system may be a host cell, and the host cell may express a fusion protein as described above. The host cell may be a eukaryotic and/or prokaryotic cell, more specifically a mammalian cell, *E. coli*, or yeast, etc, more specifically HEK293, or CHO, etc.

A fifth aspect of the present disclosure provides a method for preparing the fusion protein provided by the first aspect of the present disclosure. Those skilled in the art can choose a suitable method to prepare the fusion protein. For example, solid-phase synthesis can be used. The method for preparing the fusion protein may include: culturing the expression system provided by the fourth aspect of the present disclosure under suitable conditions to express the fusion protein, and then isolating and purifying to provide the fusion protein.

A sixth aspect of the present disclosure provides a pharmaceutical composition, which includes: the fusion protein provided by the first aspect of the present disclosure, or culture of the expression system provided by the fourth aspect of the present disclosure. The pharmaceutical composition may further include a pharmaceutically acceptable carrier. The carriers may include various excipients and diluents that are not themselves essential active ingredients and are not unduly toxic upon application. Suitable carriers should be well known to those skilled in the art, for example, a full discussion of pharmaceutically acceptable carriers can be found in Remington's Pharmaceutical Sciences (Mack Pub. Co., N.J., 1991).

A seventh aspect of the present disclosure provides the use of the fusion protein provided by the first aspect of the present disclosure, or the pharmaceutical composition provided by the sixth aspect of the present disclosure, in the preparation of a drug which may be selected from drugs for the treatment of metabolism-related diseases. The metabolism-related disease may be metabolic syndrome, and features of the metabolic syndrome can often include the following risk factors (e.g., include more than three): (1) abdominal obesity (excessive fatty tissue in or around the abdomen); (2) atherogenic dyslipidemia, or dyslipidemia including high triglycerides, low HDL cholesterol and high LDL cholesterol, which enhance the accumulation of plaque in the arterial wall; (3) elevated blood pressure; (4) insulin resistance or glucose intolerance; (5) thromboid state, such as high fibrin or plasminogen activator inhibitor-1 in blood; and (6) pro-inflammatory state, such as elevated C-reactive protein in the blood. Other risk factors may include aging, hormone imbalance and genetic factors. In a specific embodiment of the present disclosure, the fusion protein provided by the present disclosure has a good glucose-lowering and weight-loss effect in mice, and thus has been shown to be usable for the treatment of obesity or diabetes. For example, the fusion protein of the present disclosure can often be used to treat diseases through mechanisms such as reducing appetite, decreasing food intake, lowering body fat levels in patients, and increasing energy expenditure.

An eighth aspect of the present disclosure provides a method of treatment, including: administering to an individual a therapeutically effective amount of a fusion protein provided by the first aspect of the present disclosure, a culture of the expression system provided by the fourth aspect of the present disclosure, or a pharmaceutical composition provided by the sixth aspect of the present disclosure.

In the present disclosure, the term "treatment" includes prophylactic, curative or palliative treatments that result in the desired pharmaceutical and/or physiological effects. The effect preferably refers to medically reducing one or more symptoms of the disease or completely eliminating the disease, or blocking or delaying the occurrence of the disease and/or reducing the risk of disease development or deterioration.

In the present disclosure, "individual" typically includes a human, non-human primate, or other mammals (e.g., dog, cat, horse, sheep, pig, cow, etc.) that may benefit from the treatment with the preparation, kit, or combination of preparations described.

In the present disclosure, "therapeutically effective amount" generally refers to an amount that can achieve the effect of treating the diseases listed above after a proper administration period.

Sequence identity herein refers to the percentage of identical residues in the sequences participating in the alignment. The sequence identity of two or more sequences may be calculated using calculation software well known in the art, such software may be obtained from NCBI.

As is known to all, incretin hormones, such as GLP-1 analogues and Exendin-4, may cause side effects such as nausea and vomiting, which are dose-related. Therefore, as long as the ideal blood glucose level can be maintained, reducing the dosage as much as possible will theoretically alleviate the patient's discomfort and side effects. In addition, the effects of FGF21 on osteoporosis and reproduction have been reported (Scand J Clin Lab Invest, 75(2):121-5, 2015; MolMetab, 5(8):690-8, 2016). Theoretically, the risk of side effects of drugs is directly proportional to the dose administered. In the field of diabetes drugs, the safety requirements of drugs are extremely high. The inventor found that the fusion protein of the present disclosure is highly effective in controlling blood glucose and weight at a low dose, and has little effect on the gastrointestinal tract, which greatly reduces the dose of administration, thus significantly reducing the risk of potential side effects.

In the cell-based biological activity assay of the fusion proteins provided by the present disclosure, the *in vitro* bioactivity assay of the GLP-1R and GCGR agonist activities of the present disclosure adopt the luciferase reporter gene detection method. The luciferase reporter gene detection method is based on the principle that GLP-1R and GCGR can activate the downstream cAMP pathway after activation. The activity assay of FGF21 and its analogues is obtained by co-transfecting FGF21 signaling pathway-related genes into the HEK293T cells, and detecting the change in fluorescence caused by the signal. According to the report of Joseph R. Chabenne et al. and Richard D. DiMarchi et al., adding a C-terminal small peptide cex (SEQ ID NO.3) of Exendin-4 at the C-terminal of Glucagon can increase the GLP-1R agonist activity from 0.7% to 1.6%, an increase of about 2 times (J Diabetes Sci Technol. 4(6): 1322-1331, 2010 and patent US9018164 B2), but the ratio of GCGR agonist activity to GLP-1R agonist activity is only about 35:1. In addition, Evers A et al. reported (J Med Chem.; 60(10):4293-4303, 2017) that after adding the cex sequence to the C-terminal of the GCG analogue, GLP-1R agonist activity decreased by about 3 times, and GCG agonist activity decreased by about 14 times (Table 2, peptides 7 and 8 in the article). That is, simply adding the C-terminal peptide cex sequence GPSSGAPPPS (SEQ ID NO. 3) from Exendin-4 at the C-terminal of natural Glucagon does not significantly increase its GLP-1R agonist activity, but would even further weaken its GLP-1R agonist activity. On the other hand, when small peptides, such as Glucagon and GLP-1, are fused with carrier fusion proteins such as F_{C} or albumin, their activity tends to be significantly reduced due to steric hindrance (J. Pept. Sci., 14: 588-595, 2008), and this activity change is unpredictable.

The inventors unexpectedly found that after fusing GCG analogues of different sequences with F_{C}, the effects on the activities of GLP-1R and GCGR were completely different. When the GCG analogue is added with a cex or similar sequence (SEQ ID NO. 2-3) at the C-terminal and then further fused to the F_{C} chain, the activity retention rate of GLP-1R agonist activity for structures containing a cex sequence is significantly increased by up to 200-fold (Table 2 in Embodiment 2) compared to GCG analogues directly fused to an F_{C} chain, but the activity retention rate of GCGR agonist activity is essentially unchanged, or even slightly decreased.

When referring to protein stability, natural Glucagon has several sensitive degradation sites, including the DPP-IV degradation site at position 2, and SRR sites at positions 16-18. Although some reports consider that Fc can improve the chemical stability and serum stability of the active protein, for the GLP-1 or Glucagon analogues in which N-terminal must be exposed, the role of Fc seems to be inconclusive. After the fusion of natural GLP-1 or Glucagon with Fc, the obvious degradation of natural GLP-1 or Glucagon can still be observed in serum under 37 °C. To improve stability, the present disclosure introduces a mutation that is resistant to protease hydrolysis at positions 16-18 on the basis of natural Glucagon; the modification at positions 27 and 28 prevents the occurrence of deamidation and oxidation of amino acids. After the fusion of the mutant with F_{C}, the stability is further improved. In stability study, the fusion proteins provided by the present disclosure, about 60% of the GCGR agonist activity can still be detected within 72 hours. In contrast, the dimer (SEQID NO.52) formed by the fusion of natural Glucagon containing cex sequence and F_{C} has hardly detected any activity.

Almost all GCGR, GLP-1R, and GIPR poly-agonists based on Oxyntomodulin and Glucagon have introduced mutations at the second position to resist DPP-IV, for example, the mutation of L-amino acid to D-amino acid (L-Ser mutated to D-Ser ), or the introduction of an unnatural amino acid such as Aib (Cell Metabolism, 24:51-62, 2016). However, in the embodiments of the present disclosure, the dimer fusion protein that preserves natural L-Ser at the second position exhibits very high serum stability, without any sign of significant degradation by DPP-IV within 24 hours, while the corresponding peptides without F_{C} fusion are rapidly hydrolyzed by DPP-IV (Table 3). Inventors of the present disclosure prepared an active protein A001L1F6 (SEQ ID NO.51) in which natural Glucagon was fused with F_{C}, and an active protein A012L1F6 (SEQ ID NO.52) in which Glucagon-cex was fused with Fc according to the report of Joseph R. Chabenne et al. The two active proteins serve as a control to verify whether F_{C} fusion did improve the stability. However, neither A001L1F6 (SEQ ID NO.51) nor A012L1F6 (SEQ ID NO.52) showed obvious signs of resistance to DPP-IV (Embodiment 4). Also, as seen in Embodiment 4, although all mutated at positions 16-18, the stability of F_{C} fusion proteins of GCG analogues varies greatly. The spatial conformation of the protein is extremely complicated. Therefore, the mutation in accordance with SEQ ID NO. 81 not only improves the internal stability of the peptide chain, but more likely changes the conformation of the interaction between the GCG analogue and the F_{C} chain, thereby further improving the stability of the N-terminal of the fusion protein. Although some reports have suggested that binding to serum albumin (such as HSA) may improve protein stability (such as liraglutide), however, if the second position is not mutated, the half-life cannot be sustained for more than 12 hours. That is, it is impossible to support the once-a-week administration frequency. The pharmacokinetic and pharmacodynamic tests have shown that the GCG analogues provided by the present disclosure are sufficient to support the once-a-week administration frequency rather than once a day as generally reported (for example, albumin-binding liraglutide). The retention of natural amino acids further reduces the risk of immunogenicity, avoids chemical conjugation and makes the preparation process easier and more convenient.

The present disclosure also performs random glucose experiments in leptin receptor-deficient Type 2 diabetic (db/db) mice. In one embodiment, a random glucose experiment is carried out in db/db mice, the fusion proteins of the present disclosure showed a significant hypoglycemic effect than liraglutide, along with a better weight loss effect.

Weight loss experiments in DIO mice are also carried out in the present disclosure. GCGR agonists have been reported to have a potential weight loss effect. However, natural Glucagon shows little therapeutic potential since it is vulnerable to degradation and has a small molecular weight. At present, Glucagon analogues are mainly used for acute hypoglycemia symptoms. Clinical reports of long-acting GCG analogues for weight loss in diabetic patients are also emerging. It is well known that obesity is one of the causes of insulin resistance in diabetic patients, and weight loss is an important indicator to evaluate a glucose-lowering drug. In addition, the fusion protein of the present disclosure causes a significant weight loss after administration to DIO mice.

The fusion protein of the present disclosure has pharmacokinetic properties that are potentially suitable for once a week or longer administration. The dose depends on the administration frequency and administration route; the age, gender, weight, and general conditions of the subjects being treated; the condition and severity of the treatment; any accompanying diseases to be treated; and other factors apparent to those skilled in the art. At the same time, according to the condition of the subject and other pathological conditions, the fusion proteins of the present disclosure may be administered or applied in combination with one or more other therapeutically active compounds or substances. For example, the above-mentioned other therapeutically active compounds available include, but are not limited to, anti-diabetic drugs, anti-hyperlipidemia drugs, anti-obesity drugs, anti-hypertensive drugs, and reagents for the treatment of complications arising from or related to diabetes.

Metabolism-related disease (e.g., metabolic syndrome) is associated with an increased risk of coronary heart disease and other conditions related to the accumulation of vascular plaque, such as stroke and peripheral vascular disease, which become atherosclerotic cardiovascular disease (ASCVD). Patients with metabolic syndrome may progress from an early insulin resistance state to fully mature Type 2 diabetes, and the risk of ASCVD would be further increased. Not to be limited to any particular theory, the relationship between insulin resistance, metabolic syndrome and vascular disease may involve one or more common pathogenesis, including insulin-stimulated vasodilation disorder, decreased availability of insulin resistance-related correlations due to increased oxidative stress, and abnormalities of adipocyte-derived hormones (such as adiponectin) (Lteif, Mather, Can. J. Cardiol. 20 (Suppl B): 66B-76B, 2004).

The fusion proteins provided by the present disclosure may be used to treat obesity. In some aspects, the fusion proteins of the present disclosure treat obesity by reducing appetite, decreasing food intake, lowering body fat level in patients, increasing energy consumption and other mechanisms.

In some potential embodiments, the fusion proteins of the present disclosure may be used for the treatment of non-alcoholic fatty liver disease (NAFLD). NAFLD refers to broad-spectrum liver diseases, ranging from simple fatty liver (fatty degeneration) to non-alcoholic steatosis hepatitis (NASH) to liver cirrhosis (irreversible advanced scarring of the liver). All stages of NAFLD have fat accumulation in liver cells. Simple fatty liver is an abnormal accumulation of certain types of fat and triglycerides in the liver cells, but there is no inflammation or scar formation. In NASH, fat accumulation is associated with varying degrees of liver inflammation (hepatitis) and scar formation (fibrosis). Inflammatory cells may destroy liver cells (hepatocyte necrosis). In the terms "Steatosis hepatitis" and "Steatosis necrosis", steatosis refers to fatty infiltration, hepatitis refers to inflammation in the liver, and necrosis refers to destroyed liver cells. NASH may eventually lead to liver scarring (fibrosis) and then result in irreversible advanced scarring (liver cirrhosis). Liver cirrhosis caused by NASH is the final and the most severe stage of NAFLD.

In summary, the fusion proteins provided by the present disclosure has an advantage of a long half-life, which can usually be considered to support a dosing frequency of once a week or longer. In addition, the fusion proteins have significant hypoglycemic and weight-loss effects, good stability *in vivo* and *in vitro* and low immunogenicity risk. Further, as the introduction of a non-natural amino acid is not required, and chemical synthesis and conjugation steps are not involved, the fusion proteins can be prepared by recombinant method; therefore, the preparation process is greatly simplified.

The embodiments of the present disclosure will be described below through exemplary embodiments. Those skilled in the art can easily understand other advantages and effects of the present disclosure according to contents disclosed by the specification. The present disclosure may also be implemented or applied through other different specific implementation modes. Various modifications or changes may be made to all details in the specification based on different points of view and applications without departing from the spirit of the present disclosure.

Before further describing the specific embodiments of the present disclosure, it is understood that the scope of the present disclosure is not limited to the specific embodiments described below; It is also to be understood that the terminology of the disclosure is used to describe the specific embodiments, and not to limit the scope of the disclosure;

When the numerical ranges are given by the embodiments, it is to be understood that the two endpoints of each numerical range and any one between the two may be selected unless otherwise stated. Unless otherwise defined, all technical and scientific terms used in the present disclosure have the same meaning as commonly understood by one skilled in the art. In addition to the specific method, equipment and material used in the embodiments, any method, equipment and material in the existing technology similar or equivalent to the method, equipment and material mentioned in the embodiments of the present disclosure may be used to realize the invention according to the grasp of the existing technology and the record of the invention by those skilled in the art.

Unless otherwise stated, the experimental methods, detection methods, and preparation methods disclosed in the present disclosure all employ conventional techniques of molecular biology, biochemistry, chromatin structure and analysis, analytical chemistry, cell culture, recombinant DNA technology in the technical field and related fields. These techniques are well described in the existing literatures. For details, see Sambrook et al. MOLECULAR CLONING: A LABORATORY MANUAL, Second edition, Cold Spring Harbor Laboratory Press, 1989 and Third edition, 2001; Ausubel et al, CURRENT PROTOCOLS IN MOLECULAR BIOLOGY, John Wiley & Sons, New York, 1987 and periodic updates; the series METHODS IN ENZYMOLOGY, Academic Press, San Diego; Wolffe, CHROMATIN STRUCTURE AND FUNCTION, Third edition, Academic Press, San Diego, 1998; METHODS IN ENZYMOLOGY, Vol. 304, Chromatin (P.M. Wassarman and A.P. Wolffe, eds.), Academic Press, San Diego, 1999; and METHODS IN MOLECULAR BIOLOGY, Vol. 119, Chromatin Protocols (P. B. Becker, ed.) Humana Press, Totowa, 1999, and the like.

### Embodiment 1

Preparation of the fusion proteins shown in Formula I:
The fusion proteins prepared in this embodiment are represented by Formula I. The specific structure is: A-L-F (Formula I). The amino acid sequences of the dimeric fusion proteins obtained by the fusion of a GCG analogue fragment (corresponding to A in Formula I), a linker peptide fragment (corresponding to L in Formula I) and F_{C} (corresponding to F in Formula I) are preferably shown in Table 1 below.

The skilled in the art can easily prepare the dimeric fusion protein using the traditional technology on the basis of knowing the amino acid sequences of the dimeric fusion proteins: due to the presence of Fc sequence, Protein A resin chromatography with high affinity and high specificity can be used for protein purification. A feasible preparation method is given here illustratively.

The preparation process is as follows:
(1) DNA sequences were designed according to the protein sequence and amino acid codon table. Polynucleotide DNA fragments were prepared corresponding to A, L and F in the recombinant proteins, respectively, and each DNA fragment can be synthesized and spliced by conventional solid-phase synthesis technology.
(2) Primers for nested PCR amplification were designed and corresponding DNA fragments of A, L, and F were spliced to obtain the target genes; PCR splicing technology (including primer design, PCR introduced mutation and enzyme digestion, etc.) is a known technology for the skilled in the art. The skilled in the art should be aware that the PCR splicing process in this embodiment is not the only method, for example, the target gene may also be obtained through gene synthesis. Target genes were cloned into mammalian cell expression vector pTT5 (Yves Durocher) and transforming into *E*. *coli* Top 10F'. Positive clones were inoculated in 500 ml LB medium after identification for overnight culture. Cells were collected by centrifugation, followed by plasmid extraction by Omega E.Z.N.A.^{®} Endo-Free Plasmid Maxi Kit or similar methods.
(3) Transfection of HEK293F cells and expression: 1.0 mg plasmid was diluted in 25 ml Freestyle 293 expression medium (Thermofisher). 3.0 mg PEI (linear, 25KD) was diluted in 25 ml Freestyle 293 expression medium, and added to plasmid solution, the mixture was incubated at room temperature for 30 minutes after mixing well. At the same time, HEK293F cells in log phase (viability> 95%) were counted. After centrifuging at 1100 rpm for 10 minutes, the supernatant was discarded. The cells were resuspended in 450 ml Freestyle 293 expression medium. The PEI-plasmid mixture was added into the HEK293F cell suspension after incubation for shake-culturing at 37 °C, 5% CO₂, and 140 RPM. After 7 hours, the Freestyle 293 expression medium was replaced with 1000 ml 293 SFM II medium (Thermofisher) and continued for culture for 7 days.
(4) Purification of the recombinant proteins: the cell culture solution was centrifuged at 8000 rpm for 10 min to obtain the supernatant, before loading to a Protein A affinity column (Bestchrom ( Shanghai) Biotechnology Co., Ltd.) pre-balanced with equilibrium solution (20mM PB, 0.5M NaCl, pH7), and eluted by 100% eluent (0.1M Gly-HCl, pH3.0) after reequilibration. Neutralization buffer (1M Tris-HCl, pH 8.0) was added to the collection tube in advance before collecting the eluted sample. Finally, the neutralization buffer was added to 1/10 of the volume of the eluted sample. Protein concentrations were detected by conventional Bradford method.
(5) Identification of the physicochemical properties of the recombinant proteins: HPLC analysis of the purified recombinant proteins showed a purity of >95%.

### Embodiment 2

*In-vitro* cell-based bioactivity assay of the fusion proteins as shown in Formula I:
*In-vitro* cell-based bioactivity assays of the fusion proteins obtained in Embodiment 1, including GLP-1R agonist activity and GCGR agonist activity.

GLP-1R agonist bioactivity assay:
The GLP-1R agonist bioactivity assay was carried out using a luciferase reporter gene detection method. The human GLP-1R gene was cloned into mammalian cell expression plasmid pCDNA3.1 to construct a recombinant expression plasmid pCDNA3.1-GLP-1R, and the full-length luciferase gene was cloned into pCRE-EGFP plasmid to replace the EGFP gene to obtain a pCRE-Luc recombinant plasmid. CHO cells were transfected with pCDNA3.1-GLP-1R and pCRE-Luc plasmids at a molar ratio of 1:10, and stably transfected expression strains were selected to obtain recombinant CHO/GLP-1R stably transfected cell strains.

The cells were cultured in a 9-cm cell dish with DMEM/F12 medium containing 10%FBS and 300µg/ml G418. When the confluence reached about 90%, the supernatant was discarded. The cells were digested in 2ml trypsin for 3min and neutralized in 2ml DMEM/F12 medium before transferring to a 15ml centrifuge tube. The tube was centrifuged at 1000rpm for 5min. Supernatant was discarded and 2ml DMEM/F12 medium containing 10% FBS and 300µg/ml G418 was added for resuspension and counting. The cells were diluted to 5×10⁵/mL with DMEM/F12 medium containing 10% FBS. Plating 100µl in each well of a 96-well plate, i.e., 5×10⁴ per well. After adherence, the cells were cultured in DMEM/F12 medium containing 0.2% FBS overnight. After discarding the supernatant of cells in 96-well plates, the purified recombinant proteins (Tables 1-2) or natural Glucagon (Hangzhou Chinese Peptide Biochemical Co., Ltd, GLUC-004) and natural GLP-1 (Hangzhou Chinese Peptide Biochemical Co., Ltd., GLUC-016B) serving as controls were added 100µl/well after diluting with DMEM/F12 medium containing 0.2% FBS to a series of specified concentrations . The cells were cultured for 6h stimulation before detection. The assay is carried out according to the instructions of Lucifersae reporter kit (Ray Biotech, Cat: 68-LuciR-S200).

GCGR agonist bioactivity assay:
The GCGR agonist bioactivity assay is carried out using a luciferase reporter gene detection method, too. The GCGR gene was cloned into mammalian cell expression plasmid pCDNA3.1 to construct a recombinant expression plasmid pCDNA3.1-GCGR. The transfected CHO cells and the stable transfected cell strains CHO/GCGR are constructed as above.

The results are shown in Table 1:

**Table 1**

| Polypeptide code | Amino acid sequence (SEQ ID NO.) | GCGR agonist activity (EC₅₀, nM) | GLP-1R agonist activity (EC₅₀, nM) |
|---|---|---|---|
| Natural Glucagon | 24 | 0.94 | 120.87 |
| Natural GLP-1 | 1 | > 1000 | 0.52 |
| A017L1F6 | 53 | 17.06 | 19.89 |
| A021L1F6 | 54 | 17.81 | 17.71 |
| A073L1F6 | 55 | 43.23 | 50.57 |
| A089L1F6 | 56 | 33.51 | 43.68 |
| A123L1F6 | 57 | 37.36 | 45.59 |
| A135L1F6 | 58 | 51.94 | 53.55 |
| A137L1F6 | 59 | 43.94 | 63.55 |
| A175L1F6 | 60 | 52.22 | 41.68 |
| A187L1F6 | 61 | 42.31 | 42.14 |
| A209L1F6 | 62 | 63.30 | 53.88 |
| A215L1F6 | 63 | 1.34 | 1.94 |
| A227L1F6 | 64 | 1.50 | 1.48 |
| A266L1F6 | 66 | 1.33 | 1.75 |
| A271L1F6 | 67 | 1.39 | 1.34 |
| A307L1F6 | 68 | 1.62 | 1.95 |
| A451L1F6 | 73 | 11.45 | 12.96 |
| A462L1F6 | 74 | 11.38 | 20.11 |
| A499L1F6 | 75 | 12.31 | 30.54 |
| A504L1F6 | 76 | 10.57 | 21.81 |
| A512L1F6 | 77 | 12.15 | 22.77 |
| C0382L1F6 | 78 | 4.84 | 3.12 |

Note: The protein numbers in Table 1 are in accordance with the following rules: polypeptide code of GCG analogue + linker peptide code + Fc code. For example, A382L1F6 indicates that a GCG analogue polypeptide A382 is fused with an IgG F_{C} (code: F6) via a linker peptide (code:L1).

In addition, other dimeric fusion proteins that do not contain the C-terminal sequence of Exendin-4, such as G232L1F6 (SEQ ID NO.82), G352L1F6 (SEQ ID NO.83), G382L1F6 (SEQ ID NO.84), G395L1F6 (SEQ ID NO.85) and G402L1F6 (SEQ ID NO.86) were detected in *in vitro* bioactivity assays (prepared and purified in the same manner as in Embodiment 1), including GLP-1R agonist and GCGR agonist bioactivity assay.

The assay results are compared with the recombinant protein of the present disclosure, as shown in Table 2:

**Table 2**

| Code of dimeric fusion protein | SEQ ID NO. | EC₅₀ (nM) | | Activity ratio ^{a} |
|---|---|---|---|---|
| | | GCGR | GLP-1R | |
| A001L1F6 | 51 | 7.98 | 380.32 | 21 |
| A012L1F6 | 52 | 8.46 | 17.89 | |
| G232L1F6 | 82 | 1.16 | 591.48 | 212 |
| A232L1F6 | 65 | 1.45 | 2.79 | |
| G352L1F6 | 83 | 1.44 | 372.30 | 219 |
| A352L1F6 | 69 | 1.44 | 1.70 | |
| G382L1F6 | 84 | 0.97 | 407.16 | 234 |
| A382L1F6 | 70 | 1.67 | 1.74 | |
| G395L1F6 | 85 | 1.03 | 406.33 | 234 |
| A395L1F6 | 71 | 1.23 | 1.74 | |
| G402L1F6 | 86 | 1.19 | 395.90 | 214 |
| A402L1F6 | 72 | 1.32 | 1.85 | |
| Glucagon | | | | 1.4^{b} |
| Glucagon Cex | | | | |
| Glucagon | | | | 2.3^{c} |
| Glucagon Cex | | | | |

Note: a is the ratio of GLP-1R agonist activity before and after the addition of the C-terminal extension peptide Cex from Exendin-4 (any one of SEQ ID NO. 2-3 in the present disclosure).

b is the ratio calculated based on the GLP-1R agonist activity data of natural Glucagon and Glucagon Cex disclosed in Table 2 of US9018164 B2.

c is the ratio calculated based on the GLP-1R agonist activity data of natural Glucagon and Glucagon Cex disclosed in Table 1 in a paper of Joseph R. Chabenne et al. (Joseph R. Chabenne et al., J Diabetes Sci Technol. 4(6): 1322-1331, 2010).

As shown in Table 2, when the GCG analogue peptide containing the C-terminal sequence of Exendin-4 according to the present disclosure is fused with F_{C} via a linker peptide to prepare a dimer, the GLP-1R agonist activity is increased by more than 200 times, while the GCGR agonist activity shows no significant change. In contrast, the ratio of A001L1F6 to A012L1F6 is only about 21.

### Embodiment 3

DPP-IV enzyme resistance of the fusion proteins as shown in Formula I:

The purified fusion protein was dissolved in 10 mM of HEPES buffer (containing 0.05 mg/ml BSA) to a final concentration of 5 µM. Recombinant DPP-IV enzyme (final concentration of 10 nM) was added and incubated at 37°C for 24 hours before detection of GCGR bioactivity. Activity retention rate = (activity after DPP-IV enzyme treatment / activity before DPP-IV enzyme treatment) × 100%.

In this Embodiment, GCG analogues with an unnatural amino acid Aib or D-Ser introduced at the second position are used as controls:
GDSerGS: H-D-Ser-QGTFTSDYSKYLDSQAAQDFVQWLMNGGPSSGAPPPS (SEQ ID NO.79);
GAibGS: H-Aib-QGTFTSDYSKYLDSQAAQDFVQWLMNGGPSSGAPPPS (SEQ ID NO.80);
A123 (SEQ ID NO.30), A137 (SEQ ID NO.32), A175 (SEQ ID NO.33), A352 (SEQ ID NO.42), A382 (SEQ ID NO.43) and A395 (SEQ ID NO .44) serve as controls for the stability experiment in this Embodiment.

The results are shown in Table 3:

**Table 3**

| Code | Activity preservation rate(%) | Code | Activity preservation rate (%) |
|---|---|---|---|
| A001L1F6 (SEQ ID NO.51) | 2.3 | A352L1F6 (SEQ ID NO.69) | 98.8 |
| A012L1F6 (SEQ ID NO.52) | 1.7 | A382L1F6 (SEQ ID NO.70) | 99.5 |
| A017L1F6 (SEQ ID NO.53) | 5.8 | A395L1F6 (SEQ ID NO.71) | 95.3 |
| A021L1F6 (SEQ ID NO.54) | 5.6 | A402L1F6 (SEQ ID NO.72) | 96.5 |
| A073L1F6 (SEQ ID NO.55) | 97.8 | A451L1F6 (SEQ ID NO.73) | 68.3 |
| A089L1F6 (SEQ ID NO.56) | 99.7 | A462L1F6 (SEQ ID NO.74) | 59.2 |
| A123L1F6 (SEQ ID NO.57) | 97.4 | A499L1F6 (SEQ ID NO.75) | 44.6 |
| A135L1F6 (SEQ ID NO.58) | 93.2 | A504L1F6 (SEQ ID NO.76) | 61.9 |
| A137L1F6 (SEQ ID NO.59) | 97.9 | A512L1F6 (SEQ ID NO.77) | 60.5 |
| A175L1F6 (SEQ ID NO.60) | 96.8 | C0382L1F6 (SEQ ID NO.78) | 34.6 |
| A187L1F6 (SEQ ID NO.61) | 96.1 | GAibGS (SEQ ID NO.80) | 98.7 |
| A209L1F6 (SEQ ID NO.62) | 97.6 | GDSerGS (SEQ ID NO.79) | 99.2 |
| A215L1F6 (SEQ ID NO.63) | 93.7 | A123 (SEQ ID NO.30) | 7.5 |
| A227L1F6 (SEQ ID NO.64) | 92.3 | A137 (SEQ ID NO.32) | 6.8 |
| A232L1F6 (SEQ ID NO.65) | 98.2 | A175 (SEQ ID NO.33) | 9.2 |
| A266L1F6 (SEQ ID NO.66) | 98.1 | A352 (SEQ ID NO.42) | 4.3 |
| A271L1F6 (SEQ ID NO.67) | 94.3 | A382 (SEQ ID NO.43) | 5.8 |
| A307L1F6 (SEQ ID NO.68) | 94.0 | A395 (SEQ ID NO.44) | 8.9 |

### Embodiment 4

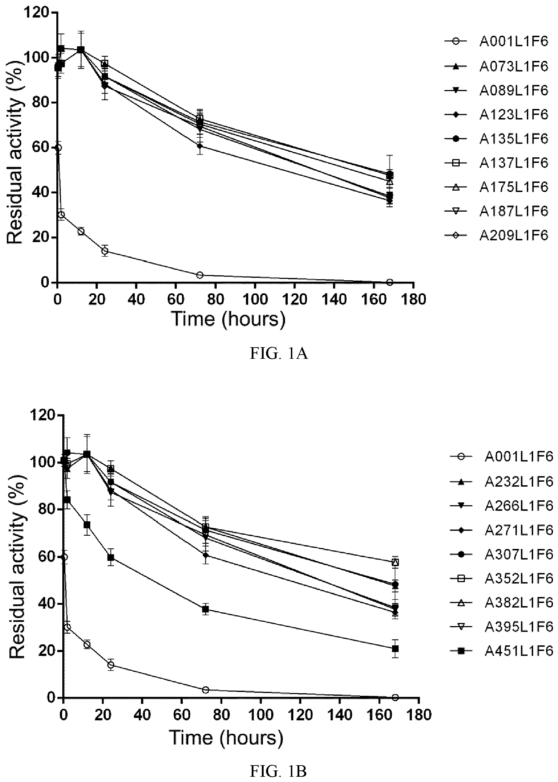

Serum stability assay of the fusion proteins as shown in Formula I:
*In-vitro* cell-based assay:
(1)Fusion proteins were concentrated by ultrafiltration and diluted with 20mM PB pH7.4 to 1.6mg/ml. After sterilization and filtration, the fusion proteins were diluted with serum (FBS, GEMINI 900108, A97E00G) by 10 times volume, then mixed and divided into sterile centrifuge tubes;
(2) Glucagon (SEQ ID NO: 24, Hangzhou Chinese Peptide Biochemical Co., Ltd, GLUC-004) was diluted to 0.2mg/ml, and further diluted with serum 10 times after sterilization and filtration, then mixed and divided into sterile centrifuge tubes;
(3) 1-2 tubes of the above samples were cryopreserved at -20 °C as controls; other tubes were placed in incubator at 37 °C, and sampled at different time points to detect GCGR agonism activity;
(4) CHO/GCGR cells were passaged twice and then plated in 96-well plates for detection of activity of samples. The relative activity over time is shown in FIGs. 1A-B. The results from FIG. 1 show that A073L1F6, A089L1F6, A123L1F6, A135L1F6, A137L1F6, A175L1F6, A187L1F6, A209L1F6, A232L1F6, A266L1F6, A271L1F6, A307L1F6, A352L1F6, A382L1F6 and A395L1F6 are significantly more stable in serum compared with other fusion proteins.

The residual activity was obtained by taking the activity at 0 hour as 100%, and comparing the values measured at the subsequent time points with that at 0 hour.

### Embodiment 5

Construction and bioactivity assay of the fusion proteins as shown in Formula II:
The fusion proteins prepared in this Embodiment are represented by Formula II. The specific structure is: A-L₁-F-L₂-B (Formula II), where A is a GCG analogue fragment, F is a long-acting protein unit fragment, B is an FGF21 analogue fragment, which may be natural FGF21 (SEQ ID NO. 87) or a derivative thereof, and L1 is a linker peptide fragment with a sequence selected from any one of SEQ ID NO.14-23. L2 is a linker peptide fragment, which may be absent or selected from any one of SEQ ID NO. 14-23. The prepared amino acids are shown in SEQ ID NO. 91-115, and the corresponding codes are shown in Table 4.

A-L₁-F-L₂-B (Formula II) can be prepared by the skilled in the art using the traditional technology on the basis of knowing the amino acid sequence. Due to the presence of F_{C} sequence, Protein A affinity chromatography with high affinity and high specificity can be used for protein purification. The specific method may refer to the preparation method in Embodiment 1. SDS-PAGE electrophoresis or amino acid sequence verification is performed on the purified recombinant protein, and the result is consistent with expectations. The natural FGF21 and FGF21 analogues may be prepared with reference to Xu J et al. (Bioconjug Chem.; 24(6):915-25, 2013) with the following modifications: the expression vector is PET30 and the host bacterium is BL21(DE3) (Merck China). Inclusion bodies are washed four times with washing solution (50 mM Tris, 150 mM NaCl, 2 M urea, pH 8.0) and weighed. Adding 1 ml of denaturing solution (50 mM Tris, 150 mM NaCl, 8 M urea, 10 mM DTT, pH 8.5) per 0.1 g of inclusion body (mass volume ratio of 1:10), gently mixing and dissolving for more than 5 h at room temperature in a shaker. The renaturation is performed by diluting at a ratio of 1:100-200. Denaturation liquid was slowly added dropwise to the renaturation liquid accompany continuous stirring; the protein-containing renaturation liquid was placed at 4 °C for 24 h after the peocess was complete. The renaturation liquid was filtered by suction filtration with 0.45 µm membrane before chromatographic purification. The specific method for preparing reference substances F9L5W and F9L5M2 can be referred to Embodiment 1.

### Construction of cells for bioactivity assay:

The puromycin resistance gene *pac* was amplified by PCR and cloned into pcDNA3.1(+) to replace the original G418 resistance gene. The GAL4DBD-ELK1, IRES, and KLB (β-klotho) genes were amplified by PCR and cloned into the pcDNA-Puro plasmid in sequence to construct the plasmid pcDNA-GAL4DBD-ELK1-IRES-KLB-Puro for cell transfection and screening. The constructed recombinant plasmid and the pFR-Luc plasmid (Agilent) were extracted by Omega E.Z.N.A.^{®} Endo-Free Plasmid Midi Kit for future use. The cell transfection process was as follows: HEK293T cells were plated 3×10⁵ cells per well in a 6-well plate and cultured overnight.

After washing the cells twice with Opti-MEM medium, 2ml Opti-MEM medium was added. Cell transfection reagent was prepared according to the following proportion: Lipofectamine 2000 (6µl): pFR-Luc (4.6µg): pcDNA-GAL4DBD-ELKl-IRES-KLB-Puro (1 µg). The cell transfection reagent, after standing for 20 min, was slowly added to the 6-well plate and gently mixed while adding. After 6 h culture, the supernatant was replaced with DMEM+10% FBS medium, and continued for culture at 37 °C with 5% CO₂. Stably transfected cell clones were screened according to FGF21 activity response.

### Cell-based bioactivity assay:

The cells were digested with pancreatin to prepare a cell suspension (1×10⁵ cells/ml, DMEM + 5% FBS + 1 µg/ml puromycin) after reaching confluence in the dish, followed by plating in a 96-well plate (100 µl per well), and culturing overnight. Samples of gradient concentrations were added and cultured for 6 h before fluorescence detection by the Luciferase Reporter Assay Kit (Lucifersae reporter kit, Ray Biotech, Cat:68-LuciR-S200). The results are shown in Table 4:

**Table 4**

| Code of active protein | Amino acid sequence (SEQ ID NO.) | GCGR agonist activity (EC₅₀, nM) | GLP-1R agonist activity (EC₅₀, nM) | FGF21 activity (EC₅₀, nM) |
|---|---|---|---|---|
| Natural Glucagon | 24 | 0.94 | 120.87 | / |
| Natural GLP-1 | 1 | > 1000 | 0.52 | / |
| Natural FGF21 | 87 | / | / | 0.12 |
| F9L5W | 116 | / | / | 0.48 |
| F6L5M2 | 117 | / | / | 0.71 |
| A012L1F9L5M2 | 118 | 10.16 | 17.89 | 0.77 |
| A352L1F8L5W | 91 | 1.22 | 1.34 | 0.55 |
| A352L1F8L5M1 | 92 | 1.20 | 1.13 | 0.79 |
| A382L1F8L5M1 | 93 | 1.20 | 1.13 | 0.79 |
| A352L1F9L5M2 | 94 | 1.30 | 1.17 | 0.83 |
| A382L1F9L5M2 | 95 | 1.16 | 1.26 | 0.77 |
| A395L1F9L5M2 | 96 | 1.34 | 1.31 | 1.36 |
| A402L1F9L5M2 | 97 | 1.25 | 1.21 | 0.89 |
| A232L1F9L5M2 | 98 | 1.17 | 1.42 | 0.85 |
| A266L1F9L5M2 | 99 | 1.37 | 1.28 | 0.93 |
| A089L1F9L5M2 | 100 | 37.51 | 45.68 | 0.87 |
| A123L1F9L5M2 | 101 | 41.25 | 51.30 | 0.74 |
| A137L1F9L5M2 | 102 | 38.13 | 47.33 | 0.82 |
| A175L1F9L5M2 | 103 | 52.21 | 42.30 | 0.71 |
| A187L1F9L5M2 | 104 | 36.43 | 40.21 | 0.84 |
| A209L1F9L5M2 | 105 | 56.11 | 51.36 | 1.67 |
| A352L1F10L5M2 | 106 | 1.15 | 1.21 | 1.45 |
| A382L1F7L5M2 | 107 | 1.35 | 1.18 | 0.81 |
| A137L1F7L5M2 | 108 | 41.38 | 37.55 | 0.71 |
| A175L1F2L5M2 | 109 | 47.34 | 54.37 | 0.69 |
| A352L1F6L5M2 | 110 | 1.46 | 1.67 | 0.85 |
| A382L1F6L5M2 | 111 | 1.34 | 1.21 | 1.54 |
| A137L1F6L5M2 | 112 | 37.69 | 51.88 | 0.79 |
| A175L1F6L5M2 | 113 | 46.27 | 48.87 | 0.73 |
| A137L1F8L5M3 | 114 | 41.20 | 59.13 | 0.82 |
| A175L1F8L5M3 | 115 | 39.20 | 47.13 | 0.63 |

Note: The protein numbers in Table 4 are in accordance with the following rules: polypeptide code of GCG analogue + linker peptide code + F_{C} code + linker peptide code + FGF21 analogue code. For example, A382L1F6L5M2 indicates that the GCG analogue polypeptide A382 is fused with an IgG F_{C} (code: F6) via a linker peptide (code:L1), and further fused with an FGF21 analogue (code: M2) via a linker peptide (code:L5) to construct a fusion protein.

### Embodiment 6

Random blood glucose test after continuous administration of fusion protein in db/db mice

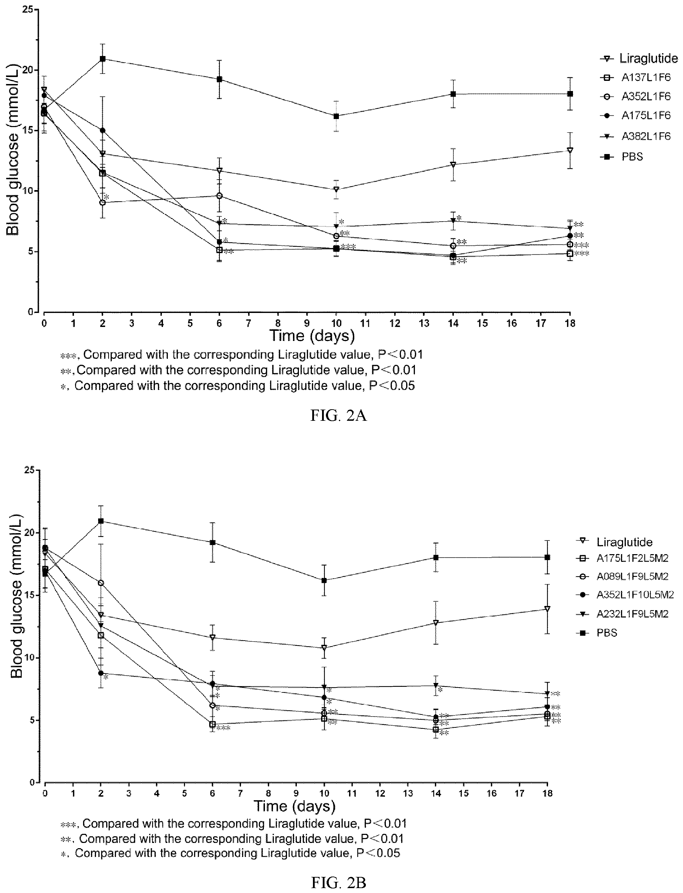

Hypoglycemic experiment in leptin receptor-deficient Type 2 diabetes (db/db) mice. The db/db mice were screened and evenly grouped according to the three indicators: body weight, non-fasting blood glucose, and OGTT response before drug administration. Each group consists of 10 mice. Individuals too large or too small were excluded as far as possible. The fusion proteins were injected subcutaneously at doses shown in Table 5. The administrations were given once every 4 days, and the first administration was at day 0 and the last one at day 16. Saline (PBS) (5 µl/g of body weight) was given to the negative control group, liraglutide (10 nmol/ kg of body weight) was given to the positive control group. The above administration to control group was given by subcutaneous injection once a day for 18 consecutive days. Random blood glucose values were measured at 9 am before the first injection and on the 2d, 6d, 10d, 14d, and 18d. The results of random blood glucose changes are shown in FIG. 2.

FIGs. 2A-2B show that the hypoglycemic effect of the fusion proteins in Table 5 is significantly superior to the positive control liraglutide in the db/db mice.

**Table 5**

| Sample | SEQ ID NO. | Dose (nmol/kg) |
|---|---|---|
| A137L1F6 | 59 | 30 |
| A175L1F6 | 60 | 30 |
| A352L1F6 | 69 | 30 |
| A382L1F6 | 70 | 30 |
| A232L1F9L5M2 | 98 | 6 |
| A089L1F9L5M2 | 100 | 6 |
| A352L1F10L5M2 | 106 | 6 |
| A175L1F2L5M2 | 109 | 6 |

### Embodiment 7

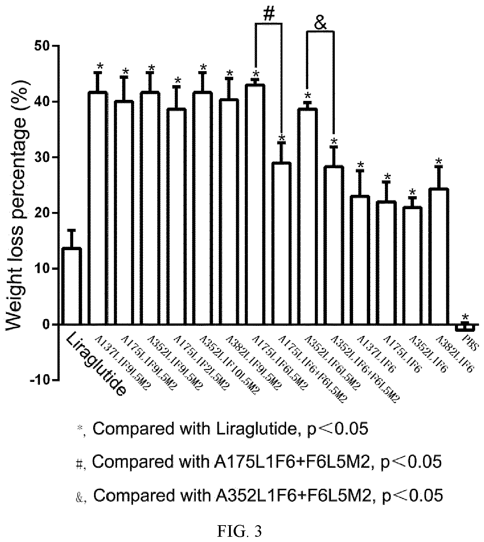

Pharmacodynamic study of continuous administration in diet-induced obese (DIO) mice:
The purpose of this Embodiment is to study the effect of different dual functional fusion proteins on the body weight of DIO mice. 7-week-old C57BL/6J male mice were fed with high-fat diet (60% kcal from fat) for another 16 weeks (a total of 23 weeks), and the test was conducted when their body weight reached approximately 55g. Feeding conditions: 12h light/12h darkness, freely fed in single cage; mice were grouped (8 mice per group) according to body weight and body weight growth curve the day before administration; at the next day, mice were administered subcutaneously. The mice are administered at a dose of 20 nmol per kg of body weight once every 4 days, with PBS sham injections on other days, for 28 continuous days. The negative control group was injected with saline (PBS) at 5 ul per kg of body weight. The positive control group was injected with liraglutide (40 nmol per kg of body weight) once a day. The body weights of the mice were measured everyday and the food intakes were recorded. Mice were sacrificed on the 5th day after the last administration. Blood was collected from the eye socket. Plasma samples were stored at -80 °C. The average body weight changes of each animal group before administration and sacrifice were calculated. The result is shown in FIG. 3.

In summary, the present disclosure effectively overcomes various shortcomings and has high industrial utilization value.

The above-mentioned embodiments are just used for exemplarily describing the principle and effects of the present disclosure instead of limiting the present disclosure. Modifications or variations of the above-described embodiments may be made by those skilled in the art without departing from the spirit and scope of the present disclosure. Therefore, all equivalent modifications or changes made by those skilled in the art without departing from the spirit and technical concept disclosed by the present disclosure shall be still covered by the claims of the present disclosure.

## Claims

1. A fusion protein, comprising a Glucagon analogue fragment and a long-acting protein unit fragment, the Glucagon analogue fragment comprising:
a) a polypeptide fragment having an amino acid sequence shown in SEQ ID No. 81:
X₁SX₃GTFTSDYSKYLDX₁₆X₁₇X₁₈AQDFVQWLX₂₇X₂₈X₂₉X'(SEQ ID No. 81);
wherein
X₁ is selected from H or Y; X₃ is selected from Q or E; X₁₆ is selected from any amino acid except Y, N, W, and H; X₁₇ is selected from any amino acid except P, L, T, F and H; X₁₈ is selected from any amino acid except P, F, H and W; X₁₇ and X₁₈ are not R at the same time; X₂₇ is selected from M or L; X₂₈ is selected from D or A; X₂₉ is T or missing; X^{z} is selected from GGPSSGAPPPS or GPSSGAPPPS;
or b) a polypeptide fragment that has an amino acid sequence having at least 90% sequence identity with SEQ ID NO. 81 and has a function of the polypeptide fragment defined in a).

2. The fusion protein according to claim 1, wherein the polypeptide fragment in a) is selected from a polypeptide fragment having an amino acid sequence shown in one of SEQ ID NO. 29, SEQ ID NO. 32, SEQ ID NO. 33, SEQ ID NO. 35, SEQ ID NO. 38, SEQ ID NO. 42, SEQ ID NO. 43, and SEQ ID NO. 44.

3. The fusion protein according to claim 1, wherein the long-acting protein unit fragment is derived from an F_{C} portion of mammalian immunoglobulin.

4. The fusion protein according to claim 3, wherein the long-acting protein unit fragment comprises:
c) a polypeptide fragment having an amino acid sequence shown in one of SEQ ID NO. 4-13; or
d) a polypeptide fragment that has an amino acid sequence having at least 90% sequence identity with one of SEQ ID NO. 4-13 and has a function of the polypeptide fragment defined in c).

5. The fusion protein according to claim 1, wherein the fusion protein further comprises a first linker peptide fragment, the first linker peptide fragment being located between the Glucagon analogue fragment and the long-acting protein unit fragment; preferably, the first linker peptide fragment is rich in G, S and/or A.

6. The fusion protein according to claim 5, wherein the first linker peptide fragment comprises a polypeptide fragment having an amino acid sequence shown in one of SEQ ID NO. 14-23.

7. The fusion protein according to claim 5, wherein the fusion protein comprises, in order from N-terminal to C-terminal, the Glucagon analogue fragment, the first linker peptide fragment and the long-acting protein unit fragment.

8. The fusion protein according to claim 1, wherein an amino acid sequence of the fusion protein is shown in one of SEQ ID NO.56, SEQ ID NO.59, SEQ ID NO.60, SEQ ID NO.62, SEQ ID NO.65, SEQ ID NO.69, SEQ ID NO.70, and SEQ ID NO.71.

9. The fusion protein according to claim 1, wherein the fusion protein further comprises an FGF21 analogue fragment.

10. The fusion protein according to claim 9, wherein the FGF21 analogue fragment comprises:
e) a polypeptide fragment having an amino acid sequence shown in SEQ ID NO. 119: 
HPIPDSSPLLQFGGQVRQX19YLYTDDAQQTEX31HLEIX36EDGTVGX43AX4
5DQSPESLLQLX56ALKPGVIQILGVKTSRFLCQRPDGALYGSLHFDPEACS
FREX98LLEDGYNVYQSEAHGLPLHX118PGNX122SPHRDPAPRGPX134RFLP
LPGLPPALPEPPGILAPQPPDVGSSDPLX167MVX170X171SQX174RSPSX179X18
0X181(SEQ ID NO. 119); wherein
 the N terminal HPIPDSSis missing or partially missing; X₁₉ is selected from R, Y, V, E orC; X₃₁ is selected from A or C; X₃₆ is selected from R or K; X₄₃ is selected from G or C; X₄₅ is selected from A, K, E or V; X₅₆ is selected from K, R, V or I; X₉₈ is selected from L, R or D; X₁₁₈ is selected from L or C; X₁₂₂ is selected from K or R; X₁₃₄ is selected from A or C; X₁₆₇ is selected from S, A or R; X₁₇₀ is selected from G or E; X₁₇₁ is selected from P or G; X₁₇₄ is selected from G, A or L; X₁₇₉ is selected from Y, A or F; X₁₈₀ is selected from A or E; Xᵢ₈ᵢ is selected from S, K or is missing;
or f) a polypeptide fragment that has an amino acid sequence having at least 80% sequence identity with SEQ ID NO. 119 and has a function of the polypeptide fragment defined in e);
preferably, the polypeptide fragment in e) is selected from a polypeptide fragment having an amino acid sequence shown in one of SEQ ID NO. 87-90.

11. The fusion protein according to claim 9, wherein the fusion protein further comprises a second linker peptide fragment, the second linker peptide fragment being located between the long-acting protein unit fragment and the FGF21 analogue fragment; preferably, the second linker peptide fragment is rich in G, S and/or A.

12. The fusion protein according to claim 11, wherein the second linker peptide fragment comprises a peptide fragment having an amino acid sequence shown in one of SEQ ID NO. 14-23.

13. The fusion protein according to claim 11, wherein the fusion protein comprises, in order from N-terminal to C-terminal, the Glucagon analogue fragment, a first linker peptide fragment, the long-acting protein unit fragment and the FGF21 analogue fragment; or, the fusion protein comprises, in order from N-terminal to C-terminal, the Glucagon analogue fragment, a first linker peptide fragment, the long-acting protein unit fragment, the second linker peptide fragment and the FGF21 analogue fragment.

14. The fusion protein according to claim 13, wherein an amino acid sequence of the fusion protein is shown in one of SEQ ID NO. 91-115.

15. An isolated polynucleotide, which encodes the fusion protein according to any one of claims 1-14.

16. A construct, comprising the isolated polynucleotide according to claim 15.

17. An expression system, wherein the expression system comprises the construct according to claim 16 or incorporates the exogenous polynucleotide according to claim 15 in the genome.

18. A method for preparing the fusion protein according to any one of claims 1-14, comprising: culturing the expression system according to claim 17 under suitable conditions to express the fusion protein, and isolating and purifying to provide the fusion protein.

19. A pharmaceutical composition, comprising the fusion protein according to any one of claims 1-14 or culture of the expression system according to claim 17.

20. The use of the fusion protein according to any one of claims 1-14 or the pharmaceutical composition according to claim 19 in the preparation of a drug.

21. The use according to claim 20, wherein the drug is selected from drugs for the treatment of metabolism-related diseases.


SEQUENCE LISTING

<110>  ZHEJIANG DOER BIOLOGICS CORPORATION

<120>  FUSION PROTEIN FOR TREATMENT OF METABOLIC DISEASE

<130>  ZDB-10167

<160>  119

<170>  SIPOSequenceListing 1.0

<210>  1
<211>  31
<212>  PRT
<213>  Artificial Sequence

<400>  1
His Ala Glu Gly Thr Phe Thr Ser Asp Val Ser Ser Tyr Leu Glu Gly
1               5                   10                  15
Gln Ala Ala Lys Glu Phe Ile Ala Trp Leu Val Lys Gly Arg Gly
            20                  25                  30

<210>  2
<211>  11
<212>  PRT
<213>  Artificial Sequence

<400>  2
Gly Gly Pro Ser Ser Gly Ala Pro Pro Pro Ser
1               5                   10

<210>  3
<211>  10
<212>  PRT
<213>  Artificial Sequence

<400>  3
Gly Pro Ser Ser Gly Ala Pro Pro Pro Ser
1               5                   10

<210>  4
<211>  232
<212>  PRT
<213>  Artificial Sequence

<400>  4
Glu Pro Lys Ser Cys Asp Lys Thr His Thr Cys Pro Pro Cys Pro Ala
1               5                   10                  15
Pro Glu Leu Leu Gly Gly Pro Ser Val Phe Leu Phe Pro Pro Lys Pro
            20                  25                  30
Lys Asp Thr Leu Met Ile Ser Arg Thr Pro Glu Val Thr Cys Val Val
        35                  40                  45
Val Asp Val Ser His Glu Asp Pro Glu Val Lys Phe Asn Trp Tyr Val
    50                  55                  60
Asp Gly Val Glu Val His Asn Ala Lys Thr Lys Pro Arg Glu Glu Gln
65                  70                  75                  80
Tyr Asn Ser Thr Tyr Arg Val Val Ser Val Leu Thr Val Leu His Gln
                85                  90                  95
Asp Trp Leu Asn Gly Lys Glu Tyr Lys Cys Lys Val Ser Asn Lys Ala
            100                 105                 110
Leu Pro Ala Pro Ile Glu Lys Thr Ile Ser Lys Ala Lys Gly Gln Pro
        115                 120                 125

Arg Glu Pro Gln Val Tyr Thr Leu Pro Pro Ser Arg Glu Glu Met Thr
    130                 135                 140
Lys Asn Gln Val Ser Leu Thr Cys Leu Val Lys Gly Phe Tyr Pro Ser
145                 150                 155                 160
Asp Ile Ala Val Glu Trp Glu Ser Asn Gly Gln Pro Glu Asn Asn Tyr
                165                 170                 175
Lys Thr Thr Pro Pro Val Leu Asp Ser Asp Gly Ser Phe Phe Leu Tyr
            180                 185                 190
Ser Lys Leu Thr Val Asp Lys Ser Arg Trp Gln Gln Gly Asn Val Phe
        195                 200                 205
Ser Cys Ser Val Met His Glu Ala Leu His Asn His Tyr Thr Gln Lys
    210                 215                 220
Ser Leu Ser Leu Ser Pro Gly Lys
225                 230

<210>  5
<211>  226
<212>  PRT
<213>  Artificial Sequence

<400>  5
Asp Lys Thr His Thr Cys Pro Pro Cys Pro Ala Pro Glu Leu Leu Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Ala Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Tyr Thr Leu Pro Pro Ser Arg Asp Glu Leu Thr Lys Asn Gln Val Ser
    130                 135                 140
Leu Thr Cys Leu Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Tyr Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly
225

<210>  6
<211>  227
<212>  PRT
<213>  Artificial Sequence

<400>  6
Glu Arg Lys Cys Cys Val Glu Cys Pro Pro Cys Pro Ala Pro Pro Val
1               5                   10                  15
Ala Gly Pro Ser Val Phe Leu Phe Pro Pro Lys Pro Lys Asp Thr Leu
            20                  25                  30
Met Ile Ser Arg Thr Pro Glu Val Thr Cys Val Val Val Asp Val Ser

35                  40                  45
His Glu Asp Pro Glu Val Gln Phe Asn Trp Tyr Val Asp Gly Val Glu
    50                  55                  60
Val His Asn Ala Lys Thr Lys Pro Arg Glu Glu Gln Phe Asn Ser Thr
65                  70                  75                  80
Phe Arg Val Val Ser Val Leu Thr Val Val His Gln Asp Trp Leu Asn
                85                  90                  95
Gly Lys Glu Tyr Lys Cys Lys Val Ser Asn Lys Gly Leu Pro Ser Ser
            100                 105                 110
Ile Glu Lys Thr Ile Ser Lys Thr Lys Gly Gln Pro Arg Glu Pro Gln
        115                 120                 125
Val Tyr Thr Leu Pro Pro Ser Arg Glu Glu Met Thr Lys Asn Gln Val
    130                 135                 140
Ser Leu Thr Cys Leu Val Lys Gly Phe Tyr Pro Ser Asp Ile Ala Val
145                 150                 155                 160
Glu Trp Glu Ser Asn Gly Gln Pro Glu Asn Asn Tyr Lys Thr Thr Pro
                165                 170                 175
Pro Met Leu Asp Ser Asp Gly Ser Phe Phe Leu Tyr Ser Lys Leu Thr
            180                 185                 190
Val Asp Lys Ser Arg Trp Gln Gln Gly Asn Val Phe Ser Cys Ser Val
        195                 200                 205
Met His Glu Ala Leu His Asn His Tyr Thr Gln Lys Ser Leu Ser Leu
    210                 215                 220
Ser Pro Gly
225

<210>  7
<211>  229
<212>  PRT
<213>  Artificial Sequence

<400>  7
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Asn Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly Lys
225

<210>  8
<211>  229
<212>  PRT
<213>  Artificial Sequence

<400>  8
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Ala Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly Lys
225

<210>  9
<211>  228
<212>  PRT
<213>  Artificial Sequence

<400>  9
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Ala Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly
225

<210>  10
<211>  228
<212>  PRT
<213>  Artificial Sequence

<400>  10
Glu Ser Lys Tyr Gly Pro Pro Cys Pro Pro Cys Pro Ala Pro Glu Ala
1               5                   10                  15
Ala Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Asn Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly
225

<210>  11
<211>  228
<212>  PRT
<213>  Artificial Sequence

<400>  11
Glu Ser Lys Tyr Gly Pro Pro Cys Pro Pro Cys Pro Ala Pro Glu Ala
1               5                   10                  15
Ala Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Ala Ser

65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly
225

<210>  12
<211>  227
<212>  PRT
<213>  Artificial Sequence

<400>  12
Asp Lys Thr His Thr Cys Pro Pro Cys Pro Ala Pro Glu Leu Leu Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Ala Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Tyr Thr Leu Pro Pro Ser Arg Asp Glu Leu Thr Lys Asn Gln Val Ser
    130                 135                 140
Leu Thr Cys Leu Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Tyr Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly Lys
225

<210>  13
<211>  227
<212>  PRT
<213>  Artificial Sequence

<400>  13
Asp Lys Thr His Thr Cys Pro Pro Cys Pro Ala Pro Glu Leu Leu Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Ala Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Tyr Thr Leu Pro Pro Ser Arg Asp Glu Leu Thr Lys Asn Gln Val Ser
    130                 135                 140
Leu Thr Cys Leu Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Tyr Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly Ala
225

<210>  14
<211>  16
<212>  PRT
<213>  Artificial Sequence

<400>  14
Gly Gly Gly Gly Ser Gly Gly Gly Gly Ser Gly Gly Gly Gly Ser Ala
1               5                   10                  15

<210>  15
<211>  12
<212>  PRT
<213>  Artificial Sequence

<400>  15
Gly Gly Ser Gly Gly Gly Ser Gly Gly Gly Gly Ser
1               5                   10

<210>  16
<211>  12
<212>  PRT
<213>  Artificial Sequence

<400>  16
Gly Gly Ser Gly Gly Gly Ala Gly Gly Gly Gly Ser
1               5                   10

<210>  17
<211>  15
<212>  PRT

<213>  Artificial Sequence

<400>  17
Gly Gly Gly Gly Ser Gly Gly Gly Gly Ser Gly Gly Gly Gly Ser
1               5                   10                  15

<210>  18
<211>  15
<212>  PRT
<213>  Artificial Sequence

<400>  18
Gly Gly Gly Gly Ala Gly Gly Gly Gly Ala Gly Gly Gly Gly Ala
1               5                   10                  15

<210>  19
<211>  16
<212>  PRT
<213>  Artificial Sequence

<400>  19
Gly Gly Ser Gly Gly Gly Gly Ser Gly Gly Gly Gly Ser Gly Gly Ala
1               5                   10                  15

<210>  20
<211>  16
<212>  PRT
<213>  Artificial Sequence

<400>  20
Gly Gly Gly Gly Ala Gly Gly Gly Gly Ala Gly Gly Gly Gly Ala Ala
1               5                   10                  15

<210>  21
<211>  5
<212>  PRT
<213>  Artificial Sequence

<400>  21
Gly Gly Gly Gly Ser
1               5

<210>  22
<211>  16
<212>  PRT
<213>  Artificial Sequence

<400>  22
Gly Gly Gly Gly Ala Gly Gly Gly Gly Ala Gly Gly Gly Gly Ala Ser
1               5                   10                  15

<210>  23
<211>  17
<212>  PRT
<213>  Artificial Sequence

<400>  23
Gly Ser Gly Gly Gly Gly Ser Gly Gly Gly Gly Ser Gly Gly Gly Gly
1               5                   10                  15
Ser

<210>  24

<211>  29
<212>  PRT
<213>  Artificial Sequence

<400>  24
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Met Asn Thr
            20                  25

<210>  25
<211>  39
<212>  PRT
<213>  Artificial Sequence

<400>  25
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Met Asn Thr Gly Pro Ser
            20                  25                  30
Ser Gly Ala Pro Pro Pro Ser
        35

<210>  26
<211>  39
<212>  PRT
<213>  Artificial Sequence

<400>  26
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Leu Ala Gly Gly Pro Ser
            20                  25                  30
Ser Gly Ala Pro Pro Pro Ser
        35

<210>  27
<211>  39
<212>  PRT
<213>  Artificial Sequence

<400>  27
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Leu Asp Gly Gly Pro Ser
            20                  25                  30
Ser Gly Ala Pro Pro Pro Ser
        35

<210>  28
<211>  39
<212>  PRT
<213>  Artificial Sequence

<400>  28
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Met Asp Gly Gly Pro Ser
            20                  25                  30
Ser Gly Ala Pro Pro Pro Ser
        35

<210>  29

<211>  39
<212>  PRT
<213>  Artificial Sequence

<400>  29
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser
        35

<210>  30
<211>  39
<212>  PRT
<213>  Artificial Sequence

<400>  30
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser
            20                  25                  30
Ser Gly Ala Pro Pro Pro Ser
        35

<210>  31
<211>  39
<212>  PRT
<213>  Artificial Sequence

<400>  31
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Met Ala Gly Gly Pro Ser
            20                  25                  30
Ser Gly Ala Pro Pro Pro Ser
        35

<210>  32
<211>  39
<212>  PRT
<213>  Artificial Sequence

<400>  32
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser
        35

<210>  33
<211>  39
<212>  PRT
<213>  Artificial Sequence

<400>  33
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser
        35

<210>  34
<211>  39
<212>  PRT
<213>  Artificial Sequence

<400>  34
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser
        35

<210>  35
<211>  39
<212>  PRT
<213>  Artificial Sequence

<400>  35
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30
Ser Gly Ala Pro Pro Pro Ser
        35

<210>  36
<211>  39
<212>  PRT
<213>  Artificial Sequence

<400>  36
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Met Asp Gly Gly Pro Ser
            20                  25                  30
Ser Gly Ala Pro Pro Pro Ser
        35

<210>  37
<211>  39
<212>  PRT
<213>  Artificial Sequence

<400>  37
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Met Ala Gly Gly Pro Ser
            20                  25                  30
Ser Gly Ala Pro Pro Pro Ser
        35

<210>  38
<211>  39
<212>  PRT
<213>  Artificial Sequence

<400>  38
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30

Ser Gly Ala Pro Pro Pro Ser
        35

<210>  39
<211>  39
<212>  PRT
<213>  Artificial Sequence

<400>  39
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser
        35

<210>  40
<211>  39
<212>  PRT
<213>  Artificial Sequence

<400>  40
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Met Ala Gly Gly Pro Ser
            20                  25                  30
Ser Gly Ala Pro Pro Pro Ser
        35

<210>  41
<211>  39
<212>  PRT
<213>  Artificial Sequence

<400>  41
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Met Asp Gly Gly Pro Ser
            20                  25                  30
Ser Gly Ala Pro Pro Pro Ser
        35

<210>  42
<211>  39
<212>  PRT
<213>  Artificial Sequence

<400>  42
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser
        35

<210>  43
<211>  39
<212>  PRT
<213>  Artificial Sequence

<400>  43
His Ser Gln Gly Thr Phe Thr Ser Asp Tyr Ser Lys Tyr Leu Asp Ser
1               5                   10                  15

Gln Ala Ala Gln Asp Phe Val Gln Trp Leu Leu Asp Gly Gly Pro Ser
            20                  25                  30
Ser Gly Ala Pro Pro Pro Ser
        35

<210>  44
<211>  39
<212>  PRT
<213>  Artificial Sequence

<400>  44
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser
        35

<210>  45
<211>  39
<212>  PRT
<213>  Artificial Sequence

<400>  45
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser
            20                  25                  30
Ser Gly Ala Pro Pro Pro Ser
        35

<210>  46
<211>  39
<212>  PRT
<213>  Artificial Sequence

<400>  46
His Ser Gln Gly Thr Phe Thr Ser Asp Tyr Ser Lys Tyr Leu Asp Glu
1               5                   10                  15
Glu Arg Ala Gln Asp Phe Val Gln Trp Leu Met Ala Thr Gly Pro Ser
            20                  25                  30
Ser Gly Ala Pro Pro Pro Ser
        35

<210>  47
<211>  39
<212>  PRT
<213>  Artificial Sequence

<400>  47
His Ser Gln Gly Thr Phe Thr Ser Asp Tyr Ser Lys Tyr Leu Asp Ser
1               5                   10                  15
Gln Arg Ala Gln Asp Phe Val Gln Trp Leu Met Asp Thr Gly Pro Ser
            20                  25                  30
Ser Gly Ala Pro Pro Pro Ser
        35

<210>  48
<211>  39
<212>  PRT
<213>  Artificial Sequence

<400>  48

His Ser Gln Gly Thr Phe Thr Ser Asp Tyr Ser Lys Tyr Leu Asp Gly
1               5                   10                  15
Gln Ala Ala Gln Asp Phe Val Gln Trp Leu Leu Asp Gly Gly Pro Ser
            20                  25                  30
Ser Gly Ala Pro Pro Pro Ser
        35

<210>  49
<211>  39
<212>  PRT
<213>  Artificial Sequence

<400>  49
His Ser Gln Gly Thr Phe Thr Ser Asp Tyr Ser Lys Tyr Leu Asp Ser
1               5                   10                  15
Glu Ala Ala Gln Asp Phe Val Gln Trp Leu Leu Ala Gly Gly Pro Ser
            20                  25                  30
Ser Gly Ala Pro Pro Pro Ser
        35

<210>  50
<211>  39
<212>  PRT
<213>  Artificial Sequence

<400>  50
His Ser Gln Gly Thr Phe Thr Ser Asp Tyr Ser Lys Tyr Leu Asp Gly
1               5                   10                  15
Glu Ala Ala Gln Asp Phe Val Gln Trp Leu Leu Asp Gly Gly Pro Ser
            20                  25                  30
Ser Gly Ala Pro Pro Pro Ser
        35

<210>  51
<211>  273
<212>  PRT
<213>  Artificial Sequence

<400>  51
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Met Asn Thr Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160
Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190

Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly

<210>  52
<211>  283
<212>  PRT
<213>  Artificial Sequence

<400>  52
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Met Asn Thr Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  53
<211>  283
<212>  PRT
<213>  Artificial Sequence

<400>  53
His Ser Gln Gly Thr Phe Thr Ser Asp Tyr Ser Lys Tyr Leu Asp Ser

1               5                   10                  15
Arg Arg Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  54
<211>  283
<212>  PRT
<213>  Artificial Sequence

<400>  54
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala

                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  55
<211>  283
<212>  PRT
<213>  Artificial Sequence

<400>  55
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Met Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  56
<211>  283
<212>  PRT
<213>  Artificial Sequence

<400>  56
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  57
<211>  283
<212>  PRT
<213>  Artificial Sequence

<400>  57
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140

Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  58
<211>  283
<212>  PRT
<213>  Artificial Sequence

<400>  58
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Met Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  59

<211>  283
<212>  PRT
<213>  Artificial Sequence

<400>  59
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  60
<211>  283
<212>  PRT
<213>  Artificial Sequence

<400>  60
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro

115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  61
<211>  283
<212>  PRT
<213>  Artificial Sequence

<400>  61
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly

275                 280

<210>  62
<211>  283
<212>  PRT
<213>  Artificial Sequence

<400>  62
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  63
<211>  283
<212>  PRT
<213>  Artificial Sequence

<400>  63
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Met Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95

Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  64
<211>  283
<212>  PRT
<213>  Artificial Sequence

<400>  64
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Met Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255

Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  65
<211>  283
<212>  PRT
<213>  Artificial Sequence

<400>  65
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  66
<211>  283
<212>  PRT
<213>  Artificial Sequence

<400>  66
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe

65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  67
<211>  283
<212>  PRT
<213>  Artificial Sequence

<400>  67
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Met Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser

225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  68
<211>  283
<212>  PRT
<213>  Artificial Sequence

<400>  68
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Met Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  69
<211>  283
<212>  PRT
<213>  Artificial Sequence

<400>  69
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45

Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  70
<211>  283
<212>  PRT
<213>  Artificial Sequence

<400>  70
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205

Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  71
<211>  283
<212>  PRT
<213>  Artificial Sequence

<400>  71
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  72
<211>  283
<212>  PRT
<213>  Artificial Sequence

<400>  72
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser

                20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  73
<211>  283
<212>  PRT
<213>  Artificial Sequence

<400>  73
His Ser Gln Gly Thr Phe Thr Ser Asp Tyr Ser Lys Tyr Leu Asp Glu
1               5                   10                  15
Glu Arg Ala Gln Asp Phe Val Gln Trp Leu Met Ala Thr Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln

180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  74
<211>  283
<212>  PRT
<213>  Artificial Sequence

<400>  74
His Ser Gln Gly Thr Phe Thr Ser Asp Tyr Ser Lys Tyr Leu Asp Ser
1               5                   10                  15
Gln Arg Ala Gln Asp Phe Val Gln Trp Leu Met Asp Thr Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  75
<211>  283
<212>  PRT
<213>  Artificial Sequence

<400>  75

His Ser Gln Gly Thr Phe Thr Ser Asp Tyr Ser Lys Tyr Leu Asp Gly
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  76
<211>  283
<212>  PRT
<213>  Artificial Sequence

<400>  76
His Ser Gln Gly Thr Phe Thr Ser Asp Tyr Ser Lys Tyr Leu Asp Ser
1               5                   10                  15
Glu Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160

Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  77
<211>  283
<212>  PRT
<213>  Artificial Sequence

<400>  77
His Ser Gln Gly Thr Phe Thr Ser Asp Tyr Ser Lys Tyr Leu Asp Gly
1               5                   10                  15
Glu Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly
        275                 280

<210>  78
<211>  274
<212>  PRT

<213>  Artificial Sequence

<400>  78
His Ser Gln Gly Thr Phe Thr Ser Asp Tyr Ser Glu Tyr Leu Asp Ser
1               5                   10                  15
Glu Arg Ala Arg Asp Phe Val Ala Trp Leu Glu Ala Gly Gly Gly Gly
            20                  25                  30
Gly Gly Ser Gly Gly Gly Gly Ser Gly Gly Gly Gly Ser Ala Glu Ser
        35                  40                  45
Lys Tyr Gly Pro Pro Cys Pro Pro Cys Pro Ala Pro Glu Phe Leu Gly
    50                  55                  60
Gly Pro Ser Val Phe Leu Phe Pro Pro Lys Pro Lys Asp Thr Leu Met
65                  70                  75                  80
Ile Ser Arg Thr Pro Glu Val Thr Cys Val Val Val Asp Val Ser Gln
                85                  90                  95
Glu Asp Pro Glu Val Gln Phe Asn Trp Tyr Val Asp Gly Val Glu Val
            100                 105                 110
His Asn Ala Lys Thr Lys Pro Arg Glu Glu Gln Phe Ala Ser Thr Tyr
        115                 120                 125
Arg Val Val Ser Val Leu Thr Val Leu His Gln Asp Trp Leu Asn Gly
    130                 135                 140
Lys Glu Tyr Lys Cys Lys Val Ser Asn Lys Gly Leu Pro Ser Ser Ile
145                 150                 155                 160
Glu Lys Thr Ile Ser Lys Ala Lys Gly Gln Pro Arg Glu Pro Gln Val
                165                 170                 175
Tyr Thr Leu Pro Pro Ser Gln Glu Glu Met Thr Lys Asn Gln Val Ser
            180                 185                 190
Leu Thr Cys Leu Val Lys Gly Phe Tyr Pro Ser Asp Ile Ala Val Glu
        195                 200                 205
Trp Glu Ser Asn Gly Gln Pro Glu Asn Asn Tyr Lys Thr Thr Pro Pro
    210                 215                 220
Val Leu Asp Ser Asp Gly Ser Phe Phe Leu Tyr Ser Arg Leu Thr Val
225                 230                 235                 240
Asp Lys Ser Arg Trp Gln Glu Gly Asn Val Phe Ser Cys Ser Val Met
                245                 250                 255
His Glu Ala Leu His Asn His Tyr Thr Gln Lys Ser Leu Ser Leu Ser
            260                 265                 270
Leu Gly


<210>  79
<211>  37
<212>  PRT
<213>  Artificial Sequence

<400>  79
Gln Gly Thr Phe Thr Ser Asp Tyr Ser Lys Tyr Leu Asp Ser Gln Ala
1               5                   10                  15
Ala Gln Asp Phe Val Gln Trp Leu Met Asn Gly Gly Pro Ser Ser Gly
            20                  25                  30
Ala Pro Pro Pro Ser
        35

<210>  80
<211>  36
<212>  PRT
<213>  Artificial Sequence

<400>  80
Gln Gly Thr Phe Thr Ser Asp Tyr Ser Lys Tyr Leu Asp Ser Gln Ala
1               5                   10                  15
Ala Gln Asp Phe Val Gln Trp Leu Met Asn Gly Gly Pro Ser Ser Gly
            20                  25                  30

Ala Pro Pro Pro
        35

<210>  81
<211>  30
<212>  PRT
<213>  Artificial Sequence

<400>  81
Xaa Ser Xaa Gly Thr Phe Thr Ser Asp Tyr Ser Lys Tyr Leu Asp Xaa
1               5                   10                  15
Xaa Xaa Ala Gln Asp Phe Val Gln Trp Leu Xaa Xaa Xaa Xaa
            20                  25                  30

<210>  82
<211>  273
<212>  PRT
<213>  Artificial Sequence

<400>  82
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160
Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190
Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly

<210>  83
<211>  273
<212>  PRT
<213>  Artificial Sequence

<400>  83

His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160
Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190
Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly


<210>  84
<211>  273
<212>  PRT
<213>  Artificial Sequence

<400>  84
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160

Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190
Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly

<210>  85
<211>  273
<212>  PRT
<213>  Artificial Sequence

<400>  85
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160
Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190
Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly

<210>  86
<211>  273
<212>  PRT

<213>  Artificial Sequence

<400>  86
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Gly Gly
            20                  25                  30
Gly Ser Gly Gly Gly Gly Ser Gly Gly Gly Gly Ser Ala Glu Ser Lys
        35                  40                  45
Tyr Gly Pro Pro Cys Pro Pro Cys Pro Ala Pro Glu Phe Leu Gly Gly
    50                  55                  60
Pro Ser Val Phe Leu Phe Pro Pro Lys Pro Lys Asp Thr Leu Met Ile
65                  70                  75                  80
Ser Arg Thr Pro Glu Val Thr Cys Val Val Val Asp Val Ser Gln Glu
                85                  90                  95
Asp Pro Glu Val Gln Phe Asn Trp Tyr Val Asp Gly Val Glu Val His
            100                 105                 110
Asn Ala Lys Thr Lys Pro Arg Glu Glu Gln Phe Ala Ser Thr Tyr Arg
        115                 120                 125
Val Val Ser Val Leu Thr Val Leu His Gln Asp Trp Leu Asn Gly Lys
    130                 135                 140
Glu Tyr Lys Cys Lys Val Ser Asn Lys Gly Leu Pro Ser Ser Ile Glu
145                 150                 155                 160
Lys Thr Ile Ser Lys Ala Lys Gly Gln Pro Arg Glu Pro Gln Val Tyr
                165                 170                 175
Thr Leu Pro Pro Ser Gln Glu Glu Met Thr Lys Asn Gln Val Ser Leu
            180                 185                 190
Thr Cys Leu Val Lys Gly Phe Tyr Pro Ser Asp Ile Ala Val Glu Trp
        195                 200                 205
Glu Ser Asn Gly Gln Pro Glu Asn Asn Tyr Lys Thr Thr Pro Pro Val
    210                 215                 220
Leu Asp Ser Asp Gly Ser Phe Phe Leu Tyr Ser Arg Leu Thr Val Asp
225                 230                 235                 240
Lys Ser Arg Trp Gln Glu Gly Asn Val Phe Ser Cys Ser Val Met His
                245                 250                 255
Glu Ala Leu His Asn His Tyr Thr Gln Lys Ser Leu Ser Leu Ser Leu
            260                 265                 270
Gly


<210>  87
<211>  181
<212>  PRT
<213>  Artificial Sequence

<400>  87
His Pro Ile Pro Asp Ser Ser Pro Leu Leu Gln Phe Gly Gly Gln Val
1               5                   10                  15
Arg Gln Arg Tyr Leu Tyr Thr Asp Asp Ala Gln Gln Thr Glu Ala His
            20                  25                  30
Leu Glu Ile Arg Glu Asp Gly Thr Val Gly Gly Ala Ala Asp Gln Ser
        35                  40                  45
Pro Glu Ser Leu Leu Gln Leu Lys Ala Leu Lys Pro Gly Val Ile Gln
    50                  55                  60
Ile Leu Gly Val Lys Thr Ser Arg Phe Leu Cys Gln Arg Pro Asp Gly
65                  70                  75                  80
Ala Leu Tyr Gly Ser Leu His Phe Asp Pro Glu Ala Cys Ser Phe Arg
                85                  90                  95
Glu Leu Leu Leu Glu Asp Gly Tyr Asn Val Tyr Gln Ser Glu Ala His
            100                 105                 110
Gly Leu Pro Leu His Leu Pro Gly Asn Lys Ser Pro His Arg Asp Pro
        115                 120                 125
Ala Pro Arg Gly Pro Ala Arg Phe Leu Pro Leu Pro Gly Leu Pro Pro

130                 135                 140
Ala Leu Pro Glu Pro Pro Gly Ile Leu Ala Pro Gln Pro Pro Asp Val
145                 150                 155                 160
Gly Ser Ser Asp Pro Leu Ser Met Val Gly Pro Ser Gln Gly Arg Ser
                165                 170                 175
Pro Ser Tyr Ala Ser
            180

<210>  88
<211>  181
<212>  PRT
<213>  Artificial Sequence

<400>  88
His Pro Ile Pro Asp Ser Ser Pro Leu Leu Gln Phe Gly Gly Gln Val
1               5                   10                  15
Arg Gln Arg Tyr Leu Tyr Thr Asp Asp Ala Gln Gln Thr Glu Ala His
            20                  25                  30
Leu Glu Ile Arg Glu Asp Gly Thr Val Gly Gly Ala Ala Asp Gln Ser
        35                  40                  45
Pro Glu Ser Leu Leu Gln Leu Lys Ala Leu Lys Pro Gly Val Ile Gln
    50                  55                  60
Ile Leu Gly Val Lys Thr Ser Arg Phe Leu Cys Gln Arg Pro Asp Gly
65                  70                  75                  80
Ala Leu Tyr Gly Ser Leu His Phe Asp Pro Glu Ala Cys Ser Phe Arg
                85                  90                  95
Glu Arg Leu Leu Glu Asp Gly Tyr Asn Val Tyr Gln Ser Glu Ala His
            100                 105                 110
Gly Leu Pro Leu His Leu Pro Gly Asn Lys Ser Pro His Arg Asp Pro
        115                 120                 125
Ala Pro Arg Gly Pro Ala Arg Phe Leu Pro Leu Pro Gly Leu Pro Pro
    130                 135                 140
Ala Leu Pro Glu Pro Pro Gly Ile Leu Ala Pro Gln Pro Pro Asp Val
145                 150                 155                 160
Gly Ser Ser Asp Pro Leu Ser Met Val Gly Gly Ser Gln Gly Arg Ser
                165                 170                 175
Pro Ser Tyr Glu Ser
            180

<210>  89
<211>  181
<212>  PRT
<213>  Artificial Sequence

<400>  89
His Pro Ile Pro Asp Ser Ser Pro Leu Leu Gln Phe Gly Gly Gln Val
1               5                   10                  15
Arg Gln Arg Tyr Leu Tyr Thr Asp Asp Ala Gln Gln Thr Glu Ala His
            20                  25                  30
Leu Glu Ile Arg Glu Asp Gly Thr Val Gly Gly Ala Ala Asp Gln Ser
        35                  40                  45
Pro Glu Ser Leu Leu Gln Leu Lys Ala Leu Lys Pro Gly Val Ile Gln
    50                  55                  60
Ile Leu Gly Val Lys Thr Ser Arg Phe Leu Cys Gln Arg Pro Asp Gly
65                  70                  75                  80
Ala Leu Tyr Gly Ser Leu His Phe Asp Pro Glu Ala Cys Ser Phe Arg
                85                  90                  95
Glu Arg Leu Leu Glu Asp Gly Tyr Asn Val Tyr Gln Ser Glu Ala His
            100                 105                 110
Gly Leu Pro Leu His Cys Pro Gly Asn Lys Ser Pro His Arg Asp Pro
        115                 120                 125
Ala Pro Arg Gly Pro Cys Arg Phe Leu Pro Leu Pro Gly Leu Pro Pro
    130                 135                 140

Ala Leu Pro Glu Pro Pro Gly Ile Leu Ala Pro Gln Pro Pro Asp Val
145                 150                 155                 160
Gly Ser Ser Asp Pro Leu Ser Met Val Gly Gly Ser Gln Gly Arg Ser
                165                 170                 175
Pro Ser Tyr Glu Ser
            180

<210>  90
<211>  177
<212>  PRT
<213>  Artificial Sequence

<400>  90
Asp Ser Ser Pro Leu Leu Gln Phe Gly Gly Gln Val Arg Gln Arg Tyr
1               5                   10                  15
Leu Tyr Thr Asp Asp Ala Gln Gln Thr Glu Ala His Leu Glu Ile Arg
            20                  25                  30
Glu Asp Gly Thr Val Gly Gly Ala Ala Asp Gln Ser Pro Glu Ser Leu
        35                  40                  45
Leu Gln Leu Lys Ala Leu Lys Pro Gly Val Ile Gln Ile Leu Gly Val
    50                  55                  60
Lys Thr Ser Arg Phe Leu Cys Gln Arg Pro Asp Gly Ala Leu Tyr Gly
65                  70                  75                  80
Ser Leu His Phe Asp Pro Glu Ala Cys Ser Phe Arg Glu Leu Leu Leu
                85                  90                  95
Glu Asp Gly Tyr Asn Val Tyr Gln Ser Glu Ala His Gly Leu Pro Leu
            100                 105                 110
His Cys Pro Gly Asn Lys Ser Pro His Arg Asp Pro Ala Pro Arg Gly
        115                 120                 125
Pro Cys Arg Phe Leu Pro Leu Pro Gly Leu Pro Pro Ala Leu Pro Glu
    130                 135                 140
Pro Pro Gly Ile Leu Ala Pro Gln Pro Pro Asp Val Gly Ser Ser Asp
145                 150                 155                 160
Pro Leu Ala Met Val Gly Pro Ser Gln Gly Arg Ser Pro Ser Tyr Ala
                165                 170                 175
Ser

<210>  91
<211>  479
<212>  PRT
<213>  Artificial Sequence

<400>  91
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val

145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Leu Leu Leu Glu Asp
385                 390                 395                 400
Gly Tyr Asn Val Tyr Gln Ser Glu Ala His Gly Leu Pro Leu His Leu
                405                 410                 415
Pro Gly Asn Lys Ser Pro His Arg Asp Pro Ala Pro Arg Gly Pro Ala
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Pro Ser Gln Gly Arg Ser Pro Ser Tyr Ala Ser
465                 470                 475

<210>  92
<211>  479
<212>  PRT
<213>  Artificial Sequence

<400>  92
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro

115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Leu
                405                 410                 415
Pro Gly Asn Lys Ser Pro His Arg Asp Pro Ala Pro Arg Gly Pro Ala
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  93
<211>  479
<212>  PRT
<213>  Artificial Sequence

<400>  93
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val

                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Leu
                405                 410                 415
Pro Gly Asn Lys Ser Pro His Arg Asp Pro Ala Pro Arg Gly Pro Ala
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  94
<211>  478
<212>  PRT
<213>  Artificial Sequence

<400>  94
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys

50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  95
<211>  478
<212>  PRT
<213>  Artificial Sequence

<400>  95
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser

20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  96
<211>  478
<212>  PRT
<213>  Artificial Sequence

<400>  96
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  97

<211>  478
<212>  PRT
<213>  Artificial Sequence

<400>  97
His Ser Gln Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460

Met Val Gly Gly Ser Gln Gly Arg Ser Pro Ser Tyr Glu Ser
465                 470                 475

<210>  98
<211>  478
<212>  PRT
<213>  Artificial Sequence

<400>  98
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430

Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  99
<211>  478
<212>  PRT
<213>  Artificial Sequence

<400>  99
His Ser Gln Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400

Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  100
<211>  478
<212>  PRT
<213>  Artificial Sequence

<400>  100
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365

Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  101
<211>  478
<212>  PRT
<213>  Artificial Sequence

<400>  101
Tyr Ser Glu Gly Thr Phe Thr Ser Asp Tyr Ser Lys Tyr Leu Asp Glu
1               5                   10                  15
Glu Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335

Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  102
<211>  478
<212>  PRT
<213>  Artificial Sequence

<400>  102
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300

Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  103
<211>  478
<212>  PRT
<213>  Artificial Sequence

<400>  103
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270

Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  104
<211>  478
<212>  PRT
<213>  Artificial Sequence

<400>  104
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240

Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  105
<211>  478
<212>  PRT
<213>  Artificial Sequence

<400>  105
Tyr Ser Glu Gly Thr Phe Thr Ser Asp Tyr Ser Lys Tyr Leu Asp Glu
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205

Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  106
<211>  478
<212>  PRT
<213>  Artificial Sequence

<400>  106
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175

Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Ala Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly
385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  107
<211>  479
<212>  PRT
<213>  Artificial Sequence

<400>  107
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Asn Ser Thr Tyr Arg Val Val Ser Val Leu Thr
    130                 135                 140

Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  108
<211>  479
<212>  PRT
<213>  Artificial Sequence

<400>  108
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110

Asn Trp Tyr Val Asp Gly Val Glu Val His Asn Ala Lys Thr Lys Pro
        115                 120                 125
Arg Glu Glu Gln Phe Asn Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  109
<211>  477
<212>  PRT
<213>  Artificial Sequence

<400>  109
   Tyr Ser Glu Gly Thr Phe Thr Ser Asp Tyr Ser Lys Tyr Leu Asp Ser
   1               5                   10                  15
   Gln Ala Ala Gln Asp Phe Val Gln Trp Leu Leu Ala Gly Gly Pro Ser
               20                  25                  30
   Ser Gly Ala Pro Pro Pro Ser Gly Gly Gly Gly Ser Gly Gly Gly Gly
           35                  40                  45
   Ser Gly Gly Gly Gly Ser Ala Asp Lys Thr His Thr Cys Pro Pro Cys
       50                  55                  60
   Pro Ala Pro Glu Leu Leu Gly Gly Pro Ser Val Phe Leu Phe Pro Pro
   65                  70                  75                  80
   Lys Pro Lys Asp Thr Leu Met Ile Ser Arg Thr Pro Glu Val Thr Cys
                   85                  90                  95
   Val Val Val Asp Val Ser His Glu Asp Pro Glu Val Lys Phe Asn Trp
               100                 105                 110
   Tyr Val Asp Gly Val Glu Val His Asn Ala Lys Thr Lys Pro Arg Glu
           115                 120                 125
   Glu Gln Tyr Ala Ser Thr Tyr Arg Val Val Ser Val Leu Thr Val Leu
       130                 135                 140
   His Gln Asp Trp Leu Asn Gly Lys Glu Tyr Lys Cys Lys Val Ser Asn
   145                 150                 155                 160
   Lys Ala Leu Pro Ala Pro Ile Glu Lys Thr Ile Ser Lys Ala Lys Gly
                   165                 170                 175
   Gln Pro Arg Glu Pro Gln Val Tyr Thr Leu Pro Pro Ser Arg Asp Glu
               180                 185                 190
   Leu Thr Lys Asn Gln Val Ser Leu Thr Cys Leu Val Lys Gly Phe Tyr
           195                 200                 205
   Pro Ser Asp Ile Ala Val Glu Trp Glu Ser Asn Gly Gln Pro Glu Asn
       210                 215                 220
   Asn Tyr Lys Thr Thr Pro Pro Val Leu Asp Ser Asp Gly Ser Phe Phe
   225                 230                 235                 240
   Leu Tyr Ser Lys Leu Thr Val Asp Lys Ser Arg Trp Gln Gln Gly Asn
                   245                 250                 255
   Val Phe Ser Cys Ser Val Met His Glu Ala Leu His Asn His Tyr Thr
               260                 265                 270
   Gln Lys Ser Leu Ser Leu Ser Pro Gly Gly Gly Gly Gly Ala Gly Gly
           275                 280                 285
   Gly Gly Ala Gly Gly Gly Gly Ala His Pro Ile Pro Asp Ser Ser Pro
       290                 295                 300
   Leu Leu Gln Phe Gly Gly Gln Val Arg Gln Arg Tyr Leu Tyr Thr Asp
   305                 310                 315                 320
   Asp Ala Gln Gln Thr Glu Ala His Leu Glu Ile Arg Glu Asp Gly Thr
                   325                 330                 335
   Val Gly Gly Ala Ala Asp Gln Ser Pro Glu Ser Leu Leu Gln Leu Lys
               340                 345                 350
   Ala Leu Lys Pro Gly Val Ile Gln Ile Leu Gly Val Lys Thr Ser Arg
           355                 360                 365
   Phe Leu Cys Gln Arg Pro Asp Gly Ala Leu Tyr Gly Ser Leu His Phe
       370                 375                 380
   Asp Pro Glu Ala Cys Ser Phe Arg Glu Arg Leu Leu Glu Asp Gly Tyr
   385                 390                 395                 400
   Asn Val Tyr Gln Ser Glu Ala His Gly Leu Pro Leu His Cys Pro Gly
                   405                 410                 415
   Asn Lys Ser Pro His Arg Asp Pro Ala Pro Arg Gly Pro Cys Arg Phe
               420                 425                 430
   Leu Pro Leu Pro Gly Leu Pro Pro Ala Leu Pro Glu Pro Pro Gly Ile
           435                 440                 445
   Leu Ala Pro Gln Pro Pro Asp Val Gly Ser Ser Asp Pro Leu Ser Met
       450                 455                 460
   Val Gly Gly Ser Gln Gly Arg Ser Pro Ser Tyr Glu Ser
   465                 470                 475

<210> 110
<211> 479
<212> PRT
<213> Artificial Sequence

<400> 110
His Ser Gln Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45

Ser Gly Gly Gly Gly Ser Ala Glu Ser Lys Tyr Gly Pro Pro Cys Pro
50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  111
<211>  479
<212>  PRT
<213>  Artificial Sequence

<400>  111
His Ser Gln Gly Thr Phe Thr Ser Asp Tyr Ser Lys Tyr Leu Asp Ser
1               5                   10                  15

Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  112
<211>  479
<212>  PRT
<213>  Artificial Sequence

<400> 112
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu
    450                 455                 460
Ser Met Val Gly Gly Ser Gln Gly Arg Ser Pro Ser Tyr Glu Ser
465                 470                 475

<210>  113
<211>  479
<212>  PRT
<213>  Artificial Sequence

<400>  113
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Phe Leu Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala His Pro Ile Pro Asp Ser
    290                 295                 300
Ser Pro Leu Leu Gln Phe Gly Gly Gln Val Arg Gln Arg Tyr Leu Tyr
305                 310                 315                 320
Thr Asp Asp Ala Gln Gln Thr Glu Ala His Leu Glu Ile Arg Glu Asp
                325                 330                 335
Gly Thr Val Gly Gly Ala Ala Asp Gln Ser Pro Glu Ser Leu Leu Gln
            340                 345                 350
Leu Lys Ala Leu Lys Pro Gly Val Ile Gln Ile Leu Gly Val Lys Thr
        355                 360                 365
Ser Arg Phe Leu Cys Gln Arg Pro Asp Gly Ala Leu Tyr Gly Ser Leu
    370                 375                 380
His Phe Asp Pro Glu Ala Cys Ser Phe Arg Glu Arg Leu Leu Glu Asp
385                 390                 395                 400
Gly Tyr Asn Val Tyr Gln Ser Glu Ala His Gly Leu Pro Leu His Cys
                405                 410                 415
Pro Gly Asn Lys Ser Pro His Arg Asp Pro Ala Pro Arg Gly Pro Cys
            420                 425                 430
Arg Phe Leu Pro Leu Pro Gly Leu Pro Pro Ala Leu Pro Glu Pro Pro
        435                 440                 445
Gly Ile Leu Ala Pro Gln Pro Pro Asp Val Gly Ser Ser Asp Pro Leu

450                 455                 460
Ser Met Val Gly Gly Ser Gln Gly Arg Ser Pro Ser Tyr Glu Ser
465                 470                 475

<210>  114
<211>  475
<212>  PRT
<213>  Artificial Sequence

<400>  114
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Asp Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala Asp Ser Ser Pro Leu Leu
    290                 295                 300
Gln Phe Gly Gly Gln Val Arg Gln Arg Tyr Leu Tyr Thr Asp Asp Ala
305                 310                 315                 320
Gln Gln Thr Glu Ala His Leu Glu Ile Arg Glu Asp Gly Thr Val Gly
                325                 330                 335
Gly Ala Ala Asp Gln Ser Pro Glu Ser Leu Leu Gln Leu Lys Ala Leu
            340                 345                 350
Lys Pro Gly Val Ile Gln Ile Leu Gly Val Lys Thr Ser Arg Phe Leu
        355                 360                 365
Cys Gln Arg Pro Asp Gly Ala Leu Tyr Gly Ser Leu His Phe Asp Pro
    370                 375                 380
Glu Ala Cys Ser Phe Arg Glu Leu Leu Leu Glu Asp Gly Tyr Asn Val
385                 390                 395                 400
Tyr Gln Ser Glu Ala His Gly Leu Pro Leu His Cys Pro Gly Asn Lys
                405                 410                 415
Ser Pro His Arg Asp Pro Ala Pro Arg Gly Pro Cys Arg Phe Leu Pro

420                 425                 430
Leu Pro Gly Leu Pro Pro Ala Leu Pro Glu Pro Pro Gly Ile Leu Ala
        435                 440                 445
Pro Gln Pro Pro Asp Val Gly Ser Ser Asp Pro Leu Ala Met Val Gly
    450                 455                 460
Pro Ser Gln Gly Arg Ser Pro Ser Tyr Ala Ser
465                 470                 475

<210>  115
<211>  475
<212>  PRT
<213>  Artificial Sequence

<400>  115
Tyr Ser Glu Gly Thr Phe Thr Ser Asp Tyr Ser Lys Tyr Leu Asp Ser
1               5                   10                  15
Gln Ala Ala Gln Asp Phe Val Gln Trp Leu Leu Ala Gly Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Glu Ser Lys Tyr Gly Pro Pro Cys Pro
    50                  55                  60
Pro Cys Pro Ala Pro Glu Ala Ala Gly Gly Pro Ser Val Phe Leu Phe
65                  70                  75                  80
Pro Pro Lys Pro Lys Asp Thr Leu Met Ile Ser Arg Thr Pro Glu Val
                85                  90                  95
Thr Cys Val Val Val Asp Val Ser Gln Glu Asp Pro Glu Val Gln Phe
            100                 105                 110
Asn Trp Tyr Val Asp Gly Val Glu Val His Asn Ala Lys Thr Lys Pro
        115                 120                 125
Arg Glu Glu Gln Phe Ala Ser Thr Tyr Arg Val Val Ser Val Leu Thr
    130                 135                 140
Val Leu His Gln Asp Trp Leu Asn Gly Lys Glu Tyr Lys Cys Lys Val
145                 150                 155                 160
Ser Asn Lys Gly Leu Pro Ser Ser Ile Glu Lys Thr Ile Ser Lys Ala
                165                 170                 175
Lys Gly Gln Pro Arg Glu Pro Gln Val Tyr Thr Leu Pro Pro Ser Gln
            180                 185                 190
Glu Glu Met Thr Lys Asn Gln Val Ser Leu Thr Cys Leu Val Lys Gly
        195                 200                 205
Phe Tyr Pro Ser Asp Ile Ala Val Glu Trp Glu Ser Asn Gly Gln Pro
    210                 215                 220
Glu Asn Asn Tyr Lys Thr Thr Pro Pro Val Leu Asp Ser Asp Gly Ser
225                 230                 235                 240
Phe Phe Leu Tyr Ser Arg Leu Thr Val Asp Lys Ser Arg Trp Gln Glu
                245                 250                 255
Gly Asn Val Phe Ser Cys Ser Val Met His Glu Ala Leu His Asn His
            260                 265                 270
Tyr Thr Gln Lys Ser Leu Ser Leu Ser Leu Gly Gly Gly Gly Gly Ala
        275                 280                 285
Gly Gly Gly Gly Ala Gly Gly Gly Gly Ala Asp Ser Ser Pro Leu Leu
    290                 295                 300
Gln Phe Gly Gly Gln Val Arg Gln Arg Tyr Leu Tyr Thr Asp Asp Ala
305                 310                 315                 320
Gln Gln Thr Glu Ala His Leu Glu Ile Arg Glu Asp Gly Thr Val Gly
                325                 330                 335
Gly Ala Ala Asp Gln Ser Pro Glu Ser Leu Leu Gln Leu Lys Ala Leu
            340                 345                 350
Lys Pro Gly Val Ile Gln Ile Leu Gly Val Lys Thr Ser Arg Phe Leu
        355                 360                 365
Cys Gln Arg Pro Asp Gly Ala Leu Tyr Gly Ser Leu His Phe Asp Pro
    370                 375                 380
Glu Ala Cys Ser Phe Arg Glu Leu Leu Leu Glu Asp Gly Tyr Asn Val

385                 390                 395                 400
Tyr Gln Ser Glu Ala His Gly Leu Pro Leu His Cys Pro Gly Asn Lys
                405                 410                 415
Ser Pro His Arg Asp Pro Ala Pro Arg Gly Pro Cys Arg Phe Leu Pro
            420                 425                 430
Leu Pro Gly Leu Pro Pro Ala Leu Pro Glu Pro Pro Gly Ile Leu Ala
        435                 440                 445
Pro Gln Pro Pro Asp Val Gly Ser Ser Asp Pro Leu Ala Met Val Gly
    450                 455                 460
Pro Ser Gln Gly Arg Ser Pro Ser Tyr Ala Ser
465                 470                 475

<210>  116
<211>  423
<212>  PRT
<213>  Artificial Sequence

<400>  116
Asp Lys Thr His Thr Cys Pro Pro Cys Pro Ala Pro Glu Leu Leu Gly
1               5                   10                  15
Gly Pro Ser Val Phe Leu Phe Pro Pro Lys Pro Lys Asp Thr Leu Met
            20                  25                  30
Ile Ser Arg Thr Pro Glu Val Thr Cys Val Val Val Asp Val Ser His
        35                  40                  45
Glu Asp Pro Glu Val Lys Phe Asn Trp Tyr Val Asp Gly Val Glu Val
    50                  55                  60
His Asn Ala Lys Thr Lys Pro Arg Glu Glu Gln Tyr Ala Ser Thr Tyr
65                  70                  75                  80
Arg Val Val Ser Val Leu Thr Val Leu His Gln Asp Trp Leu Asn Gly
                85                  90                  95
Lys Glu Tyr Lys Cys Lys Val Ser Asn Lys Ala Leu Pro Ala Pro Ile
            100                 105                 110
Glu Lys Thr Ile Ser Lys Ala Lys Gly Gln Pro Arg Glu Pro Gln Val
        115                 120                 125
Tyr Thr Leu Pro Pro Ser Arg Asp Glu Leu Thr Lys Asn Gln Val Ser
    130                 135                 140
Leu Thr Cys Leu Val Lys Gly Phe Tyr Pro Ser Asp Ile Ala Val Glu
145                 150                 155                 160
Trp Glu Ser Asn Gly Gln Pro Glu Asn Asn Tyr Lys Thr Thr Pro Pro
                165                 170                 175
Val Leu Asp Ser Asp Gly Ser Phe Phe Leu Tyr Ser Lys Leu Thr Val
            180                 185                 190
Asp Lys Ser Arg Trp Gln Gln Gly Asn Val Phe Ser Cys Ser Val Met
        195                 200                 205
His Glu Ala Leu His Asn His Tyr Thr Gln Lys Ser Leu Ser Leu Ser
    210                 215                 220
Pro Gly Lys Gly Gly Gly Gly Ala Gly Gly Gly Gly Ala Gly Gly Gly
225                 230                 235                 240
Gly Ala His Pro Ile Pro Asp Ser Ser Pro Leu Leu Gln Phe Gly Gly
                245                 250                 255
Gln Val Arg Gln Arg Tyr Leu Tyr Thr Asp Asp Ala Gln Gln Thr Glu
            260                 265                 270
Ala His Leu Glu Ile Arg Glu Asp Gly Thr Val Gly Gly Ala Ala Asp
        275                 280                 285
Gln Ser Pro Glu Ser Leu Leu Gln Leu Lys Ala Leu Lys Pro Gly Val
    290                 295                 300
Ile Gln Ile Leu Gly Val Lys Thr Ser Arg Phe Leu Cys Gln Arg Pro
305                 310                 315                 320
Asp Gly Ala Leu Tyr Gly Ser Leu His Phe Asp Pro Glu Ala Cys Ser
                325                 330                 335
Phe Arg Glu Leu Leu Leu Glu Asp Gly Tyr Asn Val Tyr Gln Ser Glu
            340                 345                 350
Ala His Gly Leu Pro Leu His Leu Pro Gly Asn Lys Ser Pro His Arg

355                 360                 365
Asp Pro Ala Pro Arg Gly Pro Ala Arg Phe Leu Pro Leu Pro Gly Leu
    370                 375                 380
Pro Pro Ala Leu Pro Glu Pro Pro Gly Ile Leu Ala Pro Gln Pro Pro
385                 390                 395                 400
Asp Val Gly Ser Ser Asp Pro Leu Ser Met Val Gly Pro Ser Gln Gly
                405                 410                 415
Arg Ser Pro Ser Tyr Ala Ser
            420

<210>  117
<211>  424
<212>  PRT
<213>  Artificial Sequence

<400>  117
Glu Ser Lys Tyr Gly Pro Pro Cys Pro Pro Cys Pro Ala Pro Glu Phe
1               5                   10                  15
Leu Gly Gly Pro Ser Val Phe Leu Phe Pro Pro Lys Pro Lys Asp Thr
            20                  25                  30
Leu Met Ile Ser Arg Thr Pro Glu Val Thr Cys Val Val Val Asp Val
        35                  40                  45
Ser Gln Glu Asp Pro Glu Val Gln Phe Asn Trp Tyr Val Asp Gly Val
    50                  55                  60
Glu Val His Asn Ala Lys Thr Lys Pro Arg Glu Glu Gln Phe Ala Ser
65                  70                  75                  80
Thr Tyr Arg Val Val Ser Val Leu Thr Val Leu His Gln Asp Trp Leu
                85                  90                  95
Asn Gly Lys Glu Tyr Lys Cys Lys Val Ser Asn Lys Gly Leu Pro Ser
            100                 105                 110
Ser Ile Glu Lys Thr Ile Ser Lys Ala Lys Gly Gln Pro Arg Glu Pro
        115                 120                 125
Gln Val Tyr Thr Leu Pro Pro Ser Gln Glu Glu Met Thr Lys Asn Gln
    130                 135                 140
Val Ser Leu Thr Cys Leu Val Lys Gly Phe Tyr Pro Ser Asp Ile Ala
145                 150                 155                 160
Val Glu Trp Glu Ser Asn Gly Gln Pro Glu Asn Asn Tyr Lys Thr Thr
                165                 170                 175
Pro Pro Val Leu Asp Ser Asp Gly Ser Phe Phe Leu Tyr Ser Arg Leu
            180                 185                 190
Thr Val Asp Lys Ser Arg Trp Gln Glu Gly Asn Val Phe Ser Cys Ser
        195                 200                 205
Val Met His Glu Ala Leu His Asn His Tyr Thr Gln Lys Ser Leu Ser
    210                 215                 220
Leu Ser Leu Gly Gly Gly Gly Gly Ala Gly Gly Gly Gly Ala Gly Gly
225                 230                 235                 240
Gly Gly Ala His Pro Ile Pro Asp Ser Ser Pro Leu Leu Gln Phe Gly
                245                 250                 255
Gly Gln Val Arg Gln Arg Tyr Leu Tyr Thr Asp Asp Ala Gln Gln Thr
            260                 265                 270
Glu Ala His Leu Glu Ile Arg Glu Asp Gly Thr Val Gly Gly Ala Ala
        275                 280                 285
Asp Gln Ser Pro Glu Ser Leu Leu Gln Leu Lys Ala Leu Lys Pro Gly
    290                 295                 300
Val Ile Gln Ile Leu Gly Val Lys Thr Ser Arg Phe Leu Cys Gln Arg
305                 310                 315                 320
Pro Asp Gly Ala Leu Tyr Gly Ser Leu His Phe Asp Pro Glu Ala Cys
                325                 330                 335
Ser Phe Arg Glu Arg Leu Leu Glu Asp Gly Tyr Asn Val Tyr Gln Ser
            340                 345                 350
Glu Ala His Gly Leu Pro Leu His Cys Pro Gly Asn Lys Ser Pro His
        355                 360                 365
Arg Asp Pro Ala Pro Arg Gly Pro Cys Arg Phe Leu Pro Leu Pro Gly

370                 375                 380
Leu Pro Pro Ala Leu Pro Glu Pro Pro Gly Ile Leu Ala Pro Gln Pro
385                 390                 395                 400
Pro Asp Val Gly Ser Ser Asp Pro Leu Ser Met Val Gly Gly Ser Gln
                405                 410                 415
Gly Arg Ser Pro Ser Tyr Glu Ser
            420

<210>  118
<211>  478
<212>  PRT
<213>  Artificial Sequence

<400>  118
His Ser Gln Gly Thr Phe Thr Ser Asp Tyr Ser Lys Tyr Leu Asp Ser
1               5                   10                  15
Arg Arg Ala Gln Asp Phe Val Gln Trp Leu Met Asn Thr Gly Pro Ser
            20                  25                  30
Ser Gly Ala Pro Pro Pro Ser Gly Gly Gly Gly Ser Gly Gly Gly Gly
        35                  40                  45
Ser Gly Gly Gly Gly Ser Ala Asp Lys Thr His Thr Cys Pro Pro Cys
    50                  55                  60
Pro Ala Pro Glu Leu Leu Gly Gly Pro Ser Val Phe Leu Phe Pro Pro
65                  70                  75                  80
Lys Pro Lys Asp Thr Leu Met Ile Ser Arg Thr Pro Glu Val Thr Cys
                85                  90                  95
Val Val Val Asp Val Ser His Glu Asp Pro Glu Val Lys Phe Asn Trp
            100                 105                 110
Tyr Val Asp Gly Val Glu Val His Asn Ala Lys Thr Lys Pro Arg Glu
        115                 120                 125
Glu Gln Tyr Ala Ser Thr Tyr Arg Val Val Ser Val Leu Thr Val Leu
    130                 135                 140
His Gln Asp Trp Leu Asn Gly Lys Glu Tyr Lys Cys Lys Val Ser Asn
145                 150                 155                 160
Lys Ala Leu Pro Ala Pro Ile Glu Lys Thr Ile Ser Lys Ala Lys Gly
                165                 170                 175
Gln Pro Arg Glu Pro Gln Val Tyr Thr Leu Pro Pro Ser Arg Asp Glu
            180                 185                 190
Leu Thr Lys Asn Gln Val Ser Leu Thr Cys Leu Val Lys Gly Phe Tyr
        195                 200                 205
Pro Ser Asp Ile Ala Val Glu Trp Glu Ser Asn Gly Gln Pro Glu Asn
    210                 215                 220
Asn Tyr Lys Thr Thr Pro Pro Val Leu Asp Ser Asp Gly Ser Phe Phe
225                 230                 235                 240
Leu Tyr Ser Lys Leu Thr Val Asp Lys Ser Arg Trp Gln Gln Gly Asn
                245                 250                 255
Val Phe Ser Cys Ser Val Met His Glu Ala Leu His Asn His Tyr Thr
            260                 265                 270
Gln Lys Ser Leu Ser Leu Ser Pro Gly Lys Gly Gly Gly Gly Ala Gly
        275                 280                 285
Gly Gly Gly Ala Gly Gly Gly Gly Ala His Pro Ile Pro Asp Ser Ser
    290                 295                 300
Pro Leu Leu Gln Phe Gly Gly Gln Val Arg Gln Arg Tyr Leu Tyr Thr
305                 310                 315                 320
Asp Asp Ala Gln Gln Thr Glu Ala His Leu Glu Ile Arg Glu Asp Gly
                325                 330                 335
Thr Val Gly Gly Ala Ala Asp Gln Ser Pro Glu Ser Leu Leu Gln Leu
            340                 345                 350
Lys Ala Leu Lys Pro Gly Val Ile Gln Ile Leu Gly Val Lys Thr Ser
        355                 360                 365
Arg Phe Leu Cys Gln Arg Pro Asp Gly Ala Leu Tyr Gly Ser Leu His
    370                 375                 380
Phe Asp Pro Glu Ala Cys Ser Phe Arg Glu Arg Leu Leu Glu Asp Gly

385                 390                 395                 400
Tyr Asn Val Tyr Gln Ser Glu Ala His Gly Leu Pro Leu His Cys Pro
                405                 410                 415
Gly Asn Lys Ser Pro His Arg Asp Pro Ala Pro Arg Gly Pro Cys Arg
            420                 425                 430
Phe Leu Pro Leu Pro Gly Leu Pro Pro Ala Leu Pro Glu Pro Pro Gly
        435                 440                 445
Ile Leu Ala Pro Gln Pro Pro Asp Val Gly Ser Ser Asp Pro Leu Ser
    450                 455                 460
Met Val Gly Gly Ser Gln Gly Arg Ser Pro Ser Tyr Glu Ser
465                 470                 475

<210>  119
<211>  181
<212>  PRT
<213>  Artificial Sequence

<400>  119
His Pro Ile Pro Asp Ser Ser Pro Leu Leu Gln Phe Gly Gly Gln Val
1               5                   10                  15
Arg Gln Xaa Tyr Leu Tyr Thr Asp Asp Ala Gln Gln Thr Glu Xaa His
            20                  25                  30
Leu Glu Ile Xaa Glu Asp Gly Thr Val Gly Xaa Ala Xaa Asp Gln Ser
        35                  40                  45
Pro Glu Ser Leu Leu Gln Leu Xaa Ala Leu Lys Pro Gly Val Ile Gln
    50                  55                  60
Ile Leu Gly Val Lys Thr Ser Arg Phe Leu Cys Gln Arg Pro Asp Gly
65                  70                  75                  80
Ala Leu Tyr Gly Ser Leu His Phe Asp Pro Glu Ala Cys Ser Phe Arg
                85                  90                  95
Glu Xaa Leu Leu Glu Asp Gly Tyr Asn Val Tyr Gln Ser Glu Ala His
            100                 105                 110
Gly Leu Pro Leu His Xaa Pro Gly Asn Xaa Ser Pro His Arg Asp Pro
        115                 120                 125
Ala Pro Arg Gly Pro Xaa Arg Phe Leu Pro Leu Pro Gly Leu Pro Pro
    130                 135                 140
Ala Leu Pro Glu Pro Pro Gly Ile Leu Ala Pro Gln Pro Pro Asp Val
145                 150                 155                 160
Gly Ser Ser Asp Pro Leu Xaa Met Val Xaa Xaa Ser Gln Xaa Arg Ser
                165                 170                 175
Pro Ser Xaa Xaa Xaa
            180